# EUROPEAN PATENT APPLICATION

(11) **EP 1 852 129 A1**
(43) Date of publication of application: **07.11.2007**
(21) Application number: 06713158.1
(22) Date of filing: 07.02.2006
(51) Int. Cl.: A61K 45/00, A61K 31/166, A61K 31/343, A61K 31/351, A61K 31/352, A61K 31/385, A61K 31/41, A61K 31/4168, A61K 31/4196, A61K 31/421, A61K 31/426, A61K 45/06, A61P 1/04, C07D 233/88, C07D 249/14, C07D 257/06, C07D 263/28, C07D 277/18, C07D 277/20, C07D 277/38, C07D 277/46

(54) **REMEDY FOR IRRITABLE BOWEL SYNDROME**

(30) Priority: 08.02.2005 JP 2005031920; 22.02.2005 JP 2005046302
(71) Applicant: Astellas Pharma Inc., Tokyo 103-8411 (JP)
(72) Inventor: AKUZAWA, Shinobu, 2-chome, Chuo-ku Tokyo, 1038411 (JP); ITO, Hiroyuki, 2-chome, Chuo-ku Tokyo, 1038411 (JP); WATANABE, Toshihiro, 2-chome, Chuo-ku Tokyo, 1038411 (JP); YAMADA, Hiroyoshi, 2-chome, Chuo-ku Tokyo, 1038411 (JP)
(74) Representative: HOFFMANN EITLE
(86) International application number: PCT/JP2006/302015
(87) International publication number: WO 2006/085510

(57) **Abstract**

The invention relates to a medicament for IBS, which comprises a dual antagonist for 5-HT_{2B} and 5-HT₇ receptors having selective binding affinities for 5-HT_{2B} and 5-HT₇ receptors.

The pharmaceutical composition of the invention is useful as a drug which is excellent in the therapeutic effect on IBS and shows lessened side effects occurring in the existing remedies for IBS, because it showed good pharmacological actions in comparison with the case of independently using a 5-HT_{2B} receptor antagonist having selective binding affinity for 5-HT_{2B} receptor or a 5-HT₇ receptor antagonist having selective binding affinity for 5-HT₇ receptor.

## Description

### TECHNICAL FIELD

This invention relates to a pharmaceutical composition which is useful as a medicament for irritable bowel syndrome.

### BACKGROUND ART

Irritable bowel syndrome (to be referred to as IBS hereinafter) is a disease in which abnormal bowel movement (diarrhea, constipation), abdominal pain, full-bowelled feeling and the like symptoms are chronically repeated due to excess movement of and secretion from the large intestine and the small intestines. Based on its symptoms, IBS is classified into a diarrhea type, a constipation type and a diarrhea-constipation alternation type. Concern has been directed toward the development of drugs having sufficient effect and safety for these symptoms, and studies on them are in progress.
Among them, a 5-HT (serotonin) regulatory drug is drawing attention as a remedy for IBS. The 5-HT is a monoamine neurotransmitter and expresses various physiological actions via 5-HT receptor. It has been pointed out that there is a causal relation between the morbid state of IBS and the amount of serotonin in blood. For example, there is a reference which pointed out that increase in the blood 5-HT concentration after a meal occurs in a patient of diarrhea type IBS, and this is deeply concerned in the morbid state (Gut (1998) 42, 42 - 46). Though it is at the clinical stage in Japan, a serotonin receptor antagonist or serotonin receptor agonist is already used in Europe and America. As a remedy for the diarrhea type, Alosetron (5-HT₃ receptor antagonist) has been used in the clinical field. However, ischemic colitis, constipation and the like side effects have been reported on said compound. In addition, as a remedy for the constipation type, Tegaserod (5-HT₄ receptor agonist) has been used in the clinical field in Europe and America, but its side effects have also been reported (Am. J. Gastroenterology (2000) 95, 2698 - 2709, Am. J. Gastroenterology (2003) 98, 750 - 758).
In recent years, pharmacological studies on other 5-HT receptor subtypes have also been carried out (Drugs, (2001) 61(3), 317 - 332). It is known that the 5-HT receptor is classified into 7 families of from 5-HT₁ to 5-HT₇, and the 5-HT₂ receptor is further divided into 3 subtypes of 5-HT_{2A}, 5-HT_{2B} and 5-HT_{2C} (Pharmacol. Rev., (1994) 46, 157 - 203). Regarding the 5-HT_{2B} receptor and 5-HT₇ receptor, there are reports pointing out on the role of said receptors in the digestive tracts. For example, there are reports stating that 5-HT_{2B} receptor is localized in human ileum longitudinal muscle, and a 5-HT_{2B} receptor antagonistic compound suppresses its contraction by 5-HT (Br. J. Pharmacol. (1995) 114, 1525 - 1527), and that the 5-HT_{2B} receptor localized in human colon is concerned in the 5-HT-induced contraction at the time of electric stimulation, and a 5-HT_{2B} receptor antagonistic compound suppresses the same (Br. J Pharmacol. (2002) 135, 1144 - 1151).
In addition, there are reports stating that 5-HT₇ receptor is present in guinea pig small intestines (Eur. J Pharmacol. (1995) 280, 243 - 250) and rat small intestines (Life Science (2001) 69, 2467 - 2475) and is concerned in the peristalsis of guinea pig ileum (Br. J. Pharmacol. (2003) 138, 1210 - 1214).

It has been reported that a 5-HT_{2B} selective antagonistic compound suppressed contraction of human gut tissue by electric stimulation (Patent Reference 1). There is a description in said reference that a 5-HT_{2B} antagonistic compound is useful in IBS. There is a report stating that a 5-HT_{2B} selective antagonistic compound suppressed contraction of rat gut tissue by serotonin stimulation (Patent Reference 2). There is a description in said reference that a 5-HT_{2B} antagonistic compound is useful in the treatment of functional bowel disorder.
On the other hand, it has been reported that a 5-HT₇ selective antagonistic compound showed its effect in a 5-HT-induced mouse defecation model and a mouse acetic acid rising model (Patent Reference 3). There is a description in said reference that a 5-HT₇ antagonistic compound is useful for the treatment and prevention of diarrhea type irritable bowel syndrome.
Though there are reports as described in the above, to date no information is available concerning the clinical confirmation of the effect of a 5-HT_{2B} receptor antagonistic compound or 5-HT₇ receptor antagonistic compound.
In addition, a patent concerning a method for treating urinary incontinence by jointly using a 5-HT_{2B} receptor selective antagonistic compound and a 5-HT₇ receptor selective antagonistic compound, or using a compound having both actions, has been opened to the public (Patent Reference 4).
However, there are no reports which disclose application to the IBS treatment by combining a 5-HT_{2B} receptor selective antagonistic compound with a 5-HT₇ receptor selective antagonistic compound or using a compound having both actions.
A patent application concerning 5-halotryptamine derivatives useful as ligands of 5-HT₆ receptor and/or 5-HT₇ receptor has been opened to the public, and compounds having binding affinity for the 3 receptors 5-HT_{2B}, 5-HT₆ and 5-HT₇ are disclosed on page 20 of said official gazette (Patent Reference 5). Various diseases in which serotonin receptors are concerned are cited in said official gazette as indications of said compounds, and there is a description as irritable bowel syndrome among them. However, there is no disclosure in said official gazette about illustrative pharmacological data showing their efficacy for irritable bowel syndrome.

Patent Reference 1: International Publication No. 02/056010
Patent Reference 2: JP-T-9-510216 (the term "JP-T" as used herein means a published Japanese translation of a PCT patent application)
Patent Reference 3: International Publication No. 04/014428
Patent Reference 4: US Patent No. 6,440,988
Patent Reference 5: International Publication No. 03/000252

### DISCLOSURE OF THE INVENTION

### PROBLEMS THAT THE INVENTION IS TO SOLVE

The object of the invention is to provide a medicament for IBS, which has excellent IBS treating effect and in which the side effects found in the conventional drugs are reduced.

### MEANS FOR SOLVING THE PROBLEMS

The present inventors have conducted intensive studies on the relationship between antagonists of 5-HT receptor subtypes and their therapeutic effect for IBS and found as a result that 5-HT_{2B} receptor and 5-HT₇ receptor are particularly important among many subtypes, that when their selective antagonistic compounds are simultaneously used, they show a strong disease-improving action which cannot be found by their single use, and further that similar effects can be verified by the use of a compound having both of the selective 5-HT_{2B} receptor and 5-HT₇ receptor antagonistic actions, thus accomplishing the invention. The medicine of the invention is characterized in that it shows antagonism for 5-HT_{2B} receptor and 5-HT₇ receptor simultaneously and selectively, and it may be a concomitant use of one receptor antagonist or a dual antagonist concomitantly having both antagonisms. This is the fact confirmed for the first time by the invention that a drug having such a characteristic receptor antagonism is useful for the treatment of IBS.
That is, the invention relates to a medicament for IBS, which comprises a selective dual antagonist for 5-HT_{2B} and 5-HT₇ receptors as an active ingredient.
More illustratively, it relates to the following embodiments of medicaments for IBS, which comprises a selective dual antagonist for 5-HT_{2B} and 5-HT₇ receptors as an active ingredient.
(1) a medicament for IBS, wherein the selective dual antagonist for 5-HT_{2B} and 5-HT₇ receptors comprises a selective dual antagonistic compound for 5-HT_{2B} and 5-HT₇ receptors having selective binding affinity for both of the 5-HT_{2B} and 5-HT₇ receptors,
(2) a medicament for IBS, wherein the selective dual antagonist for 5-HT_{2B} and 5-HT₇ receptors comprises a) a selective dual antagonistic compound for 5-HT_{2B} receptor having selective binding affinity for 5-HT_{2B} receptor as a first component and b) a selective dual antagonistic compound for 5-HT₇ receptor having selective binding affinity for 5-HT₇ receptor as a second component, and
(3) a combination product for treating IBS, which consists of a), as a first pharmaceutical preparation, a pharmaceutical preparation comprising an antagonistic compound for 5-HT_{2B} receptor having selective binding affinity for 5-HT_{2B} receptor as an active ingredient and b), as a second pharmaceutical preparation, a pharmaceutical preparation comprising an antagonistic compound for 5-HT₇ receptor having selective binding affinity for 5-HT₇ receptor as an active ingredient, wherein said first and second pharmaceutical preparations are administered simultaneously or separately.

Also, the invention relates to a medicament for IBS, wherein the "dual antagonistic compound for 5-HT_{2B} and 5-HT₇ receptors having selective binding affinity for both of the 5-HT_{2B} and 5-HT₇ receptors" described in the aforementioned (1) is a fluorene derivative represented by the following general formula (I) or a salt thereof. 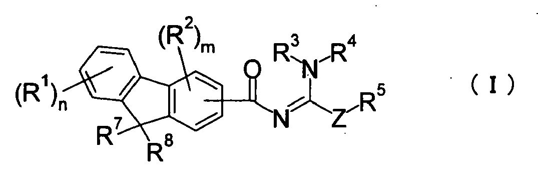 wherein the symbols have the following significances.
R¹ and R² are the same or different from each other and each represents -R⁰, lower alkenyl, lower alkynyl, halogen, -OH, -O-R⁰, -O-CO-R⁰, -NH₂, -NR⁶-R⁰, -CN, -NO₂, -CHO, -CONH₂, -CO-NR⁶-R⁰, -CO₂H, -CO₂-R⁰, -CO-R⁰, -NR⁶-CO-R⁰, -NR⁶-CO₂-R⁰, -O-CO-NR⁶-R⁰, -SH, -S(O)ₚ-R⁰, -S(O)₂-NH₂, -S(O)₂-NR⁶-R⁰, -NR⁶-S(O)₂-R⁰, -R⁰⁰-O-CO-R⁰, -R⁰⁰-NR⁶-R⁰, -R⁰⁰-CN, -R⁰⁰-CONH₂, -R⁰⁰-CO-NR⁶-R⁰, -R⁰⁰-CO₂H, -R⁰⁰-CO₂-R⁰, -R⁰⁰-CO-R⁰, -R⁰⁰-NR⁶-CO-R⁰, -R⁰⁰-NR⁶-CO₂-R⁰ -R⁰⁰-O-CO-NR⁶-R⁰, cycloalkyl or nitrogen-containing saturated heterocyclic ring; wherein the nitrogen-containing saturated heterocyclic ring may be substituted with 1 to 2 substituents selected from the group consisting of lower alkyl, -OH, -O-R⁰, -NH₂, -NR⁶-R⁰ and oxo (=0);
R° is the same or different from each other and represents a lower alkyl which may be substituted with 1 or more substituents selected from the group consisting of -OH, -O-C₁₋₄ alkyl, -NH₂, -NR⁶-C₁₋₄ alkyl and halogen;
R⁶ is the same or different from one another and represents lower alkyl or H;
R⁰⁰ is the same or different from one another and represents lower alkylene;
p is 0, 1 or 2;
n is 0, 1 or 2;
m is 0 or 1;
R⁷ and R⁸ are the same or different from each other and each represents -H, -R°, halogen,
-OH, -O-R⁰, -NH₂, -NR⁶-R⁰, -NR⁶-CO-R⁰, -O-R⁰⁰-OH, -O-R⁰⁰-O-R⁰, cycloalkyl, nitrogen-containing saturated heterocyclic ring, or R⁷ and R⁸ may be taken together to form a group selected from the group consisting of oxo (=O), =N-OH, =N-OR⁰ and tetrahydropyranylidene, or R⁷ and R⁸ may be taken together to form a lower alkylene which may be interrupted by 1 to 2 groups selected from the group consisting of -O-, -S(O)p-, -NR⁶- and -CONR⁶-, and may form a 3- to 8-membered ring together with the C atom to which they are attached.
Z is -NH-;
R³ is -H or R°; and
R⁴ and R⁵ are the same or different from each other and each represents -H, -R⁰, -CO₂-R⁰, -CO-R⁰, or R⁴ and R⁵ may be taken together to form a divalent group, which may form a 5-membered heterocyclic ring together with the -N-C-Z- group to which R⁴ and
R⁵ are attached, wherein Z may further be -O- or S- and the 5-membered ring may be substituted with 1 or 2 substituents selected from lower alkyl, -OH, -O-R⁰, -NH₂,
-NR⁶-R⁰, and oxo (=O); the same is applied hereinafter.

In addition, the invention also includes the following embodiments.
[1] Use of a selective dual antagonist for 5-HT_{2B} and 5-HT₇ receptors, for producing a medicament for IBS.
[2] Use of a "antagonistic compound for 5-HT_{2B} receptor having selective binding affinity for 5-HT_{2B} receptor", for producing a medicament for IBS comprising a selective dual antagonist for 5-HT_{2B} and 5-HT₇ receptors as an active ingredient.
[3] Use of a "antagonistic compound for 5-HT₇ receptor having selective binding affinity for 5-HT₇ receptor", for producing a medicament for IBS comprising a selective dual antagonist for 5-HT_{2B} and 5-HT₇ receptors as an active ingredient.
[4] A method for treating IBS, which comprises administering an effective amount of a selective dual antagonist for 5-HT_{2B} and 5-HT₇ receptors to a patient.
[5] A method for treating IBS, which comprises administering a combination product consisting of a pharmaceutical preparation comprising a 5-HT_{2B} selective antagonistic compound as an active ingredient and a pharmaceutical preparation comprising a 5-HT₇ selective antagonistic compound as an active ingredient, simultaneously or separately to a patient.
[6] An IBS-treating effect improving agent for a patient undergoing administration of a "antagonistic compound for 5-HT₇ receptor having selective binding affinity for 5-HT₇ receptor" for the treatment of IBS, which comprises a "antagonistic compound for 5-HT_{2B} receptor having selective binding affinity for 5-HT_{2B} receptor" as an active ingredient.
[7] An IBS-treating effect improving agent for a patient undergoing administration of a "5-HT_{2B} receptor antagonistic compound having selective binding affinity for 5-HT_{2B} receptor" for the treatment of IBS, which comprises a "antagonistic compound for 5-HT₇ receptor having selective binding affinity for 5-HT₇ receptor" as an active ingredient.

### EFFECTS OF THE INVENTION

Since the active ingredient of the medicine of the invention inhibits both functions of the 5-HT_{2B} and 5-HT₇ receptors, it exerts excellent IBS treating effect which cannot be attained by a selective antagonist of one of the receptors. In addition, since a broad range of side effects caused by the antagonisms for receptors other than the 5-HT_{2B} and 5-HT₇ receptors are also reduced, it is useful as an IBS treating drug having excellent effects and high safety.

### BRIEF DESCRIPTION OF THE DRAWINGS

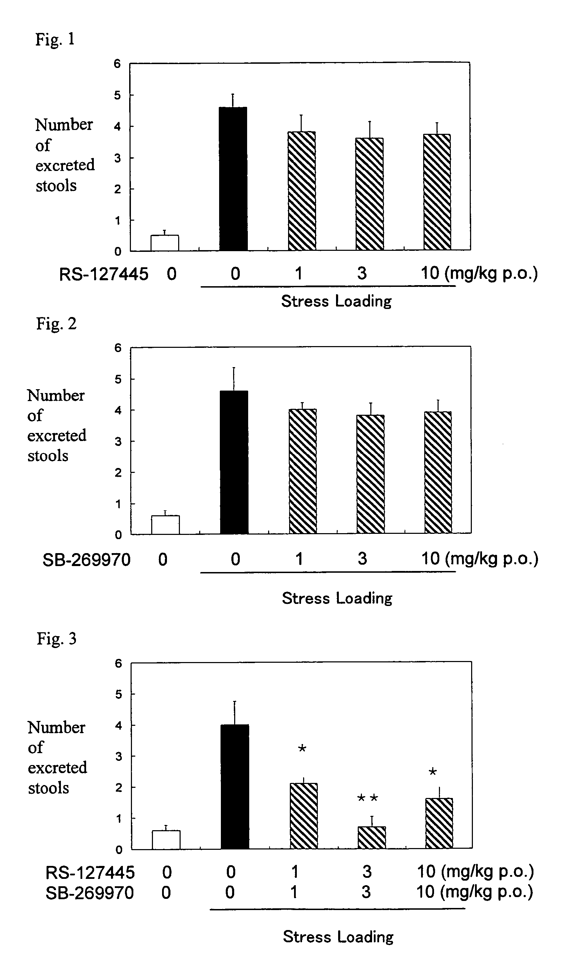

Fig. 1 is a graph showing a result of measurement of the number of excreted stools at the time of the RS-127445 administration, in the rat restraint stress-induced defecation model of Inventive Example 1. Significant difference was not found in each of the 1, 3 and 10 mg/kg administration groups in comparison with the non-administration group (N = 10).
Fig. 2 is a graph showing a result of measurement of the number of excreted stools at the time of the SB-269970 administration, in the rat restraint stress-induced defecation model of Inventive Example 1. Significant difference was not found in each of the 1, 3 and 10 mg/kg administration groups in comparison with the non-administration group (N = 10).
Fig. 3 is a graph showing a result of measurement of the number of excreted stools at the time of the RS-127445 and SB-269970 simultaneous administration, in the rat restraint stress-induced defecation model of Inventive Example 1. Statistical test was carried out by Dunnett's t-test. In the graph, * indicates that it is significant at a significance level of 5%, and ** that it is significant at 1%, and *** that it is significant at 0.1%, respectively (N = 10).

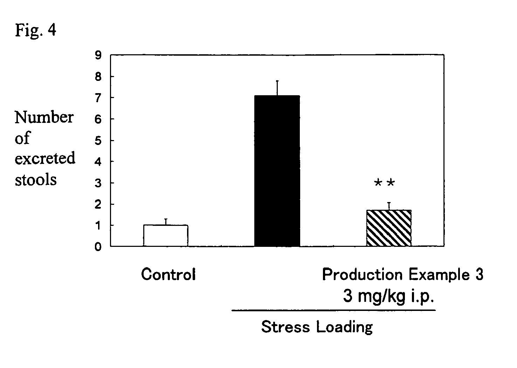

Fig. 4 is a graph showing a result of measurement of the number of excreted stools at the time of administration of the compound of Production Example 3, in the rat restraint stress-induced defecation model of Inventive Example 1. Statistical test was carried out by Dunnett's t-test. In the graph, *** indicates that it is significant at a significance level of 0.1 % (N = 10).

### BEST MODE FOR CARRYING OUT THE INVENTION

The following describes the invention in detail.
The term "antagonist" means a drug which acts antagonistically on an agonist to reduce the effect. In the present invention, a "dual antagonist for 5-HT_{2B} and 5-HT₇ receptors" means a drug which acts antagonistically with serotonin to reduce simultaneously the effect mediated by both of the 5-HT_{2B} and 5-HT₇ receptors, and includes pharmaceutical preparations containing as an active ingredient a compound having both of the antagonistic actions, and pharmaceutical preparations containing two active ingredients, i.e., a compound having a 5-HT_{2B} receptor antagonistic action and a compound having a 5-HT₇ receptor antagonistic action.
The term "binding affinity" means the ability capable of binding a part of a receptor, and it can be assessed by comparing the Ki values calculated by an in vitro receptor-binding test as described below in Test Example, or in some cases by comparing the IC₅₀ values in the receptor-binding test conducted in the parallel condition. In this context, when the IC₅₀ value cannot be calculated because a sufficient inhibitory effect is not indicated at a given concentration in the receptor-binding test, the IC₅₀ value is sometimes regarded as over the concentration.
The terms "antagonistic compound for 5-HT_{2B} receptor", "antagonistic compound for 5-HT₇ receptor" and "dual antagonistic compound for 5-HT_{2B} and 5-HT₇ receptors" include preferably antagonistic compounds in which the receptor binding affinity Ki values in the respectively selectively binding receptors are 1 µM or lower, more preferably 0.5 µM or lower, even more preferably 0.1 µM or lower, and particularly 0.05 µM or lower.

When the binding affinity for a certain receptor is more "selective" than that of other receptors, it means that the binding affinity for said receptor is higher than that of "other receptors". In the invention, the term "selective" means that the Ki value or IC₅₀ value indicating the binding affinity for said receptor is one-tenth or less, preferably one-fiftieth or less, more preferably one-hundredth or less, even more preferably one-five hundredth or less, particularly one-thousandth or less in comparison with that of "other receptors".
In this context, "other receptor" includes other receptors reported in the existing non-selective serotonin antagonists, which are particularly involved in unfavorable effects.
Thus, the "antagonistic compound for 5-HT_{2B} receptor having a selective binding affinity for 5-HT_{2B} receptor (hereinafter abbreviated to 5-HT_{2B} selective antagonistic compound)" includes specifically those selective against α₁, M₁ and D₂ receptors, preferably against α₁, M₁, D₂, 5-HT_{1A}, 5-HT_{1B}, 5-HT₄, 5-HT₆ and 5-HT₇ receptors, more preferably against α₁, M₁, D₂, 5-HT_{1A}, 5-HT_{1B}, 5-HT_{2A}, 5-HT_{2C}, 5-HT₃, 5-HT₄, 5-HT₆ and 5-HT₇ receptors.
The "antagonistic compound for 5-HT₇ receptor having a selective binding affinity for 5-HT₇ receptor (hereinafter abbreviated to 5-HT₇ selective antagonistic compound)" includes those selective against the α₁, M₁ and D₂ receptors, preferably against α₁, M₁, D₂, 5-HT_{1A}, 5-HT_{1B}, 5-HT_{2B}, 5-HT₃, 5-HT₄ and 5-HT₆ receptors, more preferably against α₁, M₁, D₂, 5-HT_{1A}, 5-HT_{1B}, 5-HT_{2A}, 5-HT_{2B}, 5-HT_{2C}, 5-HT₃, 5-HT₄ and 5-HT₆ receptors.
The "dual antagonistic compound for 5-HT_{2B} and 5-HT₇ receptors having a selective binding affinity for both of the 5-HT_{2B} and 5-HT₇ receptors (hereinafter abbreviated to 5-HT_{2B} and 5-HT₇ selective dual antagonistic compound)" includes those selective against the α₁, M₁ and D₂ receptors, preferably against α₁, M₁, D₂, 5-HT_{1A}, 5-HT_{1B}, 5-HT₃, 5-HT₄ and 5-HT₆ receptors, more preferably against α₁, M₁, D₂, 5-HT_{1A}, 5-HT_{1B}, 5-HT_{2A}, 5-HT_{2C}, 5-HT₃, 5-HT₄ and 5-HT₆ receptors.
In addition, the "a selective dual antagonist for 5-HT_{2B} and 5-HT₇ receptors" includes "combined preparations comprising a 5-HT_{2B} selective antagonistic compound and a 5-HT₇ selective antagonistic compound" or "a dual antagonist for the 5-HT_{2B} and 5-HT₇ receptors comprising as an active ingredient a 5-HT_{2B} and 5-HT₇ selective dual antagonistic compound ", in which the binding affinity (Ki value or IC₅₀ value) for the 5-HT_{2B} and 5-HT₇ receptors is one-tenth or less, preferably one-fiftieth or less, more preferably one-hundredth or less, even more preferably one-five hundredth or less, and particularly one-thousandth or less, to the α₁, M₁ and D₂ receptors, preferably to α₁, M₁, D₂, 5-HT_{1A}, 5-HT_{1B}, 5-HT₃, 5-HT₄ and 5-HT₆ receptors, more preferably to α₁, M₁, D₂, 5-HT_{1A}, 5-HT_{1B}, 5-HT_{2A}, 5-HT_{2C}, 5-HT₃, 5-HT₄ and 5-HT₆ receptors.

The "5-HT_{2B} selective antagonistic compound", "5-HT₇ selective antagonistic compound" and "5-HT_{2B} and 5-HT₇ selective dual antagonistic compound" can easily be found by screening a large amount of compounds to determine receptor affinity according to a method as shown in Reference Examples. In this evaluation, an HTS (high-throughput screening) method is employed as a routine and efficient means. As for the test compounds employed in screening, new synthetic compounds, commercially available products or known compounds registered in chemical libraries on which a variety of activities are unknown, and a group of compounds obtained by a combinatorial chemical technique, can be used. In addition, naturally occurring products derived from culture supernatants of microorganisms, plants or marine organisms, or animal tissue extracts, and the like can be employed. In addition, chemically modified compounds derived from compounds found in screening can be used.
For example, the "5-HT_{2B} selective antagonistic compound" according to the invention can be found out by carrying out the receptor affinity screening method described in the Reference Examples 1 and 3 which are described later, or a method similar to this. As illustrative compounds, for example, RS-127445 (British Journal of Pharmacology (1999) 127, 1075 - 1082), LY-266097 (*J Serotonin Res.* (1996) 3, 131), SB-200646 *(*J. Med. Chem. (1993) 36, 1104), SB-204741 *(*J Med. Chem. (1995) 38, 855), SB-20.6553 *(*J Med. Chem. (1996) 39, 2773), SB-215505 (British J Pharm. (2004) 142, 1332 - 1342), SB-221284 (9^{th} RSC-SCI Medicinal Chemistry Symposium (1997) P1 (Poster), 7 Sep), EGIS-7625 (Cardiovascular Drugs and Therapy (2003) 17, 427 - 434), PGN-1164 (Digestive Disease Week ASGE Annual Meeting, Orlando (2003) Abs T-1797), PRX-08066 (Daily Drug News. com (Daily Essentials) Dec 5 (2005)), 4-(thio or selenoxanthen-9-ylidene)piperidine or acridine derivative (WO 2003035646, US 2003166672), 2-oxazole amine derivative (WO 2003068226), 2-thiazole amine derivative (WO 2003068227) and the like conventionally known 5-HT_{2B} selective antagonistic compounds can be cited, though not limited thereto and other 5-HT_{2B} receptor-selective compounds can also be included therein. Particularly preferred are the aforementioned RS-127445, LY-266097, SB-204741, SB-215505, SB-206553), EGIS-7625, PGN-1164, 4-(thio or selenoxanthen-9-ylidene)piperidine or acridine derivative, 2-oxazole amine derivative (WO 2003068226) and 2-thiazole amine derivative.
The "5-HT₇ selective antagonistic compound" according to the invention can be found out, for example, by carrying out the receptor affinity screening method described in the Reference Examples 2 and 3 which are described later, or a method similar to this. As illustrative compounds, for example, DR-4004 (J. Med. Chem. (1999) 42, 533), SB-258719 (J. Med. Chem. (1998) 41, 654 - 657), SB-258741 (J. Med. Chem. (2000) 43, 342 - 345), SB-269970 *(*J Med. Chem. (2000) 43, 342 - 345), SB-656104 (Bioorg. Med. Chem. Lett. (2002) 12, 3341 - 3344), SB-691673 (Bioorg. Med. Chem. Lett. (2003) 13, 1055 - 1058), an aminotriazole derivative (Bioorg. Med. Chem. Lett. (2004) 14, 4245 - 4248), an aminotetralin derivative *(*J Med. Chem. (2004) 47, 3927 - 3930), an aminochroman derivative *(*J Med. Chem. (2004) 47, 3927 - 3930), an 11-phenylapomorphine derivative (J. Med. Chem. (2001) 44, 1337 - 1340), an ergorine derivative (WO 9632944), a tetrahydrobenzoindole derivative (Bioorg. Med. Chem. Lett. (2002) 12, 2549 - 2552), an aminoalkylpyrrolidine derivative (WO 200236560), an amidourea derivative (WO 200236554), a sulfone derivative (US 20040229864), a sulfonamide (WO 2005005387) and the like conventionally known 5-HT₇ selective antagonistic compounds can be cited, though not limited thereto and other 5-HT₇ receptor-selective compounds can also be included therein. Particularly preferred are the aforementioned DR-4004, SB-258719, SB-258741, SB-269970, SB-656104, SB-691673, aminotriazole derivative, aminotetralin derivative, 11-phenylapomorphine derivative, tetrahydrobenzoindole derivative, aminoalkylpyrrolidine derivative, amidourea derivative and sulfone derivative.

In addition, the "5-HT_{2B} and 5-HT₇ selective dual antagonistic compound" according to the invention can be found out, for example, by successively carrying out the receptor affinity screening methods described in the Reference Examples 1 to 3 which are described later. Preferred are compounds represented by the aforementioned formula (I) or salts thereof, and particularly preferred are the following compounds or salts thereof.
(i) A compound in which R³ is -H or R⁰ and R⁴ and R⁵ are -H or R°, more preferably a compound in which every one of R³, R⁴ and R⁵ is -H.
(ii) A compound in which n is 1 and R¹ is lower alkyl substituted with a group selected from the class consisting of -OH, -O-C₁₋₄ alkyl, -NR⁶-C₁₋₄ alkyl and halogen, or halogen, -OH, -O-R⁰, -NH₂, -CN, -CHO or -NO₂, or a compound in which n is 0.
(iii) A compound in which m is 0, or a compound in which m is 1 and R² is -R° or halogen.
(iv) A compound in which R⁷ and R⁸ are the same or different from each other and each represents -H, -R°, -OH, -O-R⁰, -O-R⁰⁰-OH or -O-R⁰⁰-O-R⁰, or R⁷ and R⁸ together form oxo group. In this case, C₁₋₃ alkyl is preferable as the R°, and C₁₋₃ alkylene as the R⁰⁰, respectively.
(v) A compound in which R⁷ and R⁸ together form "lower alkylene which may be discontinued with 1 or 2 divalent groups selected from the class consisting of -O-, -S(O)p-, -NR⁶- and -CONR⁶-" and form a 3- to 8-membered ring together with the C atoms to which they are bonded.

In the definition of the general formula in this specification, the term "lower" means a straight or branched carbon chain having from 1 to 6 carbon atoms (to be referred to as C₁₋₆ hereinafter) unless otherwise noted. Accordingly, the "lower alkyl" is a C₁₋₆ alkyl, preferably methyl, ethyl, propyl, isopropyl, butyl, isobutyl or tert-butyl.
The "lower alkenyl" includes C₂₋₆ alkenyl groups, preferably, vinyl, allyl, 1-propenyl, isopropenyl, 1-butenyl, 2-butenyl and 3-butenyl. The "lower alkynyl" includes C₂₋₆ alkynyl groups, preferably, ethynyl, 1-propynyl, 2-propynyl, 1-butynyl, 2-butynyl, 3-butynyl and 1-methyl-2-propynyl.
The "lower alkylene" includes, preferably, straight chain alkylene such as methylene, ethylene, trimethylene, tetramethylene and the like, and branched alkylene such as methylmethylene. Methylene, ethylene and trimethylene are particularly preferred.
The "halogen" means F, Cl, Br or I.
The "cycloalkyl" means C₃₋₁₀ cycloalkyl group which may have a bridge, and includes preferably cyclopropyl, cyclopentyl, cyclohexyl groups and adamantyl.
The "nitrogen-containing saturated heterocyclic ring" means a 5- to 8-membered saturated or partially unsaturated monocyclic heterocycle which contain one N atom and may contain an additional heteroatom selected from N, S and O, and includes preferably, pyrrolidinyl, piperidinyl, piperazinyl, azepanyl, diazepanyl, morpholinyl, thiomorpholinyl and tetrahydropyridyl groups.
The "oxygen-containing saturated heterocyclic ring" means a 5- to 8-membered saturated or partially unsaturated monocyclic heterocycle which contain one O atom and may contain an additional N atom, and includes preferably, tetrahydrofuranyl, tetrahydropyranyl, dihydropyranyl and morpholinyl groups.

The term "which may be substituted" indicates "unsubstituted" or "having 1 to 5 identical or different substituents".
For example, the term "lower alkyl which may be substituted with halogen" means the above-mentioned lower alkyl and lower alkyl substituted with one or more halogens, preferably C₁₋₂ alkyl having 1 to 5 F atoms, more preferably fluoromethyl, difluoromethyl, trifluoromethyl.
The term "lower alkylene which may be interrupted by 1 to 2 groups selected from the group consisting of -O-, -S(O)ₚ-, -NR⁶- and -CONR⁶-" means a lower alkylene or lower alkylene into which 1 or 2 groups selected from the group consisting of -O-, -S(O)p-, -NR⁶- and -CONR⁶- is inserted at the internal or terminal position. For example, it is exemplified by -(CH₂)₂-, -(CH₂)₃-, -(CH₂)₄-, -(CH₂)₃-O-, -(CH₂)₂-O-(CH₂)₂-, -(CH₂)₂-S(O)-(CH₂)₂-, -(CH₂)₂-N(CH₃)-(CH₂)₂-, -O-(CH₂)₂-O-, -S-(CH₂)₂-S-, -CH₂-S-CH₂-, or CH₂CONHCH₂-, preferably by -(CH₂)₄-, -S-(CH₂)₂-S-, -(CH₂)₂-O-(CH₂)₂-, or -(CH₂)₃-O-.

The salts of the compounds as active ingredients in the drugs of the invention are pharmaceutically acceptable salts, specifically including acid addition salts with inorganic acids such as hydrochloric acid, hydrobromic acid, hydroiodic acid, sulfuric acid, nitric acid, phosphoric acid, and the like, or with organic acids such as formic acid, acetic acid, propionic acid, oxalic acid, malonic acid, succinic acid, fumaric acid, maleic acid, lactic acid, malic acid, tartaric acid, citric acid, methanesulfonic acid, ethanesulfonic acid, aspartic acid, glutamic acid, and the like. In addition, salts with inorganic bases including metals such as sodium, potassium, magnesium, calcium, aluminum, and the like, or organic bases such as methylamine, ethylamine, ethanolamine, lysine, ornithine, and the like, and ammonium salts are included.

In some cases, the compound as an active ingredient in the drugs of the invention exists as a geometrical isomer or tautomer. For example, the following tautomers exist in a compound of the above-mentioned formula (I) in which R³ is -H. 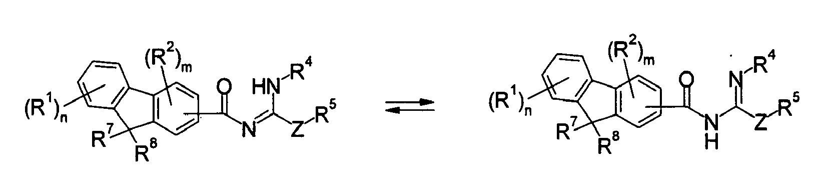 The active ingredient in the drugs of the present invention includes one of such tautomers or a mixture thereof. In addition, when an isomer exist on the basis of an asymmetric carbon atom or atoms, the active ingredient in the drugs of the invention includes these optical isomers in a form of mixture or isolated form. In some cases, N-oxides may be formed depending on the type of the substituents; such N-oxide derivatives are included in the invention. In addition, a variety of their hydrates or solvates and polymorphic substances are also included.
In the compounds as active ingredients in the drugs of the invention, some compounds are metabolized in the living body and converted into the compounds of formula (I) or their salts; such compounds, so-called prodrugs, are also included in the invention. As the groups for forming prodrugs, those described in Prog. Med. 5:2157-2161 (1985) or in "Iyakuhin no Kaihatsu (Development of Medicines)" vol.7, Bunshi Sekkey (Molecular Design) 163-198, published in 1990, Hirokawa Publishing Company, are exemplified.

Typical processes for producing the compounds as active ingredients in the drugs of the invention will be illustrated as follows. The 5-HT_{2B} selective antagonistic compounds and 5-HT₇ selective antagonistic compounds may be produced referring to the following documents.
(5-HT_{2B} selective antagonistic compounds); RS-127445 (British Journal of Pharmacology (1999) 127, 1075 - 1082), LY-266097 (J. Serotonin Res. (1996) 3, 131), SB-200646 (J. Med. Chem. (1993) 36, 1104), SB-204741 (J. Med. Chem. (1995) 38, 855), SB-206553 (J. Med. Chem. (1996) 39, 2773), EGIS-7625 (Cardiovascular Drugs and Therapy (2003) 17, 427 - 434), 4-(thio or selenoxanthen-9-ylidene)piperidine or acridine derivative (WO 2003035646, US 2003166672), 2-oxazole amine derivative (WO 2003068226), 2-thiazole amine derivative (WO 2003068227).
(5-HT₇ selective antagonistic compounds); DR-4004 (J. Med. Chem. (1999) 42, 533), SB-258719 (J. Med. Chem. (1998) 41, 654 - 657), SB-258741 *(*J Med. Chem. (2000) 43, 342 - 345), SB-269970 (J. Med. Chem. (2000) 43, 342 - 345), SB-656104 (Bioorg. Med. Chem. Lett. (2002) 12, 3341 - 3344), SB-691673 (Bioorg. Med. Chem. Lett. (2003) 13, 1055 - 1058), an aminotriazole derivative (Bioorg. Med. Chem. Lett. (2004) 14, 4245 - 4248), an aminotetralin derivative (J. Med. Chem. (2004) 47, 3927 - 3930), an aminochroman derivative (J. Med. Chem. (2004) 47, 3927 - 3930), an 11-phenylapomorphine derivative (J. Med. Chem. (2001) 44, 1337 - 1340), an ergorine derivative (WO 9632944), a tetrahydrobenzoindole derivative (Bioorg. Med. Chem. Lett. (2002) 12, 2549 - 2552), an aminoalkylpyrrolidine derivative (WO 200236560), an amidourea derivative (WO 200236554), a sulfone derivative (US 20040229864), a sulfonamide (WO 2005005387).

### (Production methods)

The compounds of the invention and pharmaceutically acceptable salts thereof may be produced by employing various conventionally known synthesis methods, making use of characteristics based on their core structure or the kind of substituent groups. In this connection, depending on the kind of functional group, it is technically effective in some cases to protect said functional group with an appropriate protecting group, or replace said functional group with a group which can be easily converted into said functional group, at a stage of starting compounds or intermediates. Examples of such a functional group include amino group, hydroxyl group, carbonyl group, carboxyl group and the like, and the protecting groups described in "Protective Groups in Organic Synthesis (3rd Edition, 1999, John Wiley & Sons)" edited by T.W. Greene and P.G. Wuts may for example be cited as their protecting groups which may be optionally used in response to the reaction conditions. According to such a method, the compound of interest may be obtained by introducing said protecting group and carrying out the reaction, and then removing the corresponding protecting group or converting it into a desired group.
In addition, similar to the case of the aforementioned protecting group, prodrugs of the compound of the invention may be produced by introducing a specific group at a stage of starting compounds or intermediates, or carrying out a reaction using the obtained compound of the invention. The reaction may be carried out by employing general esterification, amidation, dehydration and the like conventional methods known to those skilled in the art.

In addition, typical production methods of the compound represented by formula (I) are described.

### (Production Method 1)

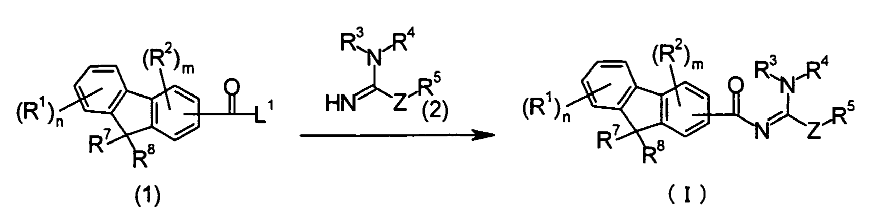

(In the formula, L¹ represents -OH or -O-lower alkyl, or halogen, -O-methanesulfonyl, -O-p-toluenesulfonyl or the like leaving group.)

The compound (I) of the invention may be produced by subjecting a compound represented by (1) which is a carboxylic acid or a reactive derivative thereof and an amine derivative (2) to an amidation reaction.
When a free carboxylic acid in which L¹ in the starting compound (1) is OH is used, a method in which the compound (1) and amine derivative (2) are dehydration condensed in the presence of a condensing agent is used. In that case, it is preferable to use N,N'-dicyclohexylcarbodiimide (DCC), 1-[3-(dimethylamino)propyl]-3-ethylcarbodiimide (WSC), 1,1'-carbonyldiimidazole (CDI), 2-(1H-benzotriazol-1-yl)-1,1,3,3-tetramethyluronium hexafluorophosphate (HBTU), diphenylphosphoryl azide (DPPA), phosphorus oxychloride or the like condensing agent and further, as occasion demands, an additive agent (e.g., N-hydroxysuccinimide (HONSu), 1-hydroxybenzotriazole (HOBt) or the like).
The reaction is carried out by using the compound (1) and amine derivative (2) in equivalent amounts or one of them in an excess amount, and using the condensing agent in equivalent amount or excess amount based on the carboxylic acid. The reaction may be carried out under cooling to heating, preferably at -20°C to 60°C, in a inert solvent such as benzene, toluene, xylene or the like aromatic hydrocarbon, dichloromethane, 1,2-dichloroethane, chloroform or the like halogenated hydrocarbon, diethyl ether, tetrahydrofuran (THF), dioxane, dimethoxyethane (DME) or the like ether, N,N-dimethylformamide (DMF), dimethyl sulfoxide (DMSO), ethyl acetate, acetonitrile, water or the like, or in a mixed solvent thereof.
When a compound in which L¹ in the starting compound (1) is a leaving group, namely a reactive derivative of the carboxylic acid, is used, the reaction is carried out using the compound (1) and amine derivative (2) in equivalent amounts or one of them in an excess amount, under the same conditions of the aforementioned case of the use of free carboxylic acid. As the reactive derivative of carboxylic acid to be used in this case, an acid halide (acid chloride, acid bromide or the like), an acid anhydride (phenyl chloroformate, a mixed anhydride prepared from p-toluenesulfonic acid or isovaleric acid or the like, or symmetric acid anhydride of itself), an activated ester (an ester which may be prepared using phenol which may be substituted with nitro group, fluorine atom or the like electron withdrawing group, HOBt, HONSu or the like), a lower alkyl ester and the like may be exemplified, and each of them may be produced from the carboxylic acid using corresponding reaction obvious to those skilled in the art. Depending on the kind of reactive derivatives, it is advantageous in some cases, for smoothly progressing the reaction, to carry out the reaction in the presence of a base (triethylamine, diisopropylethylamine, N-methylmorpholine, pyridine, 4-(N,N-dimethylamino)pyridine or the like organic base, or sodium bicarbonate or the like inorganic base or the like). Pyridine may also serve as the solvent. In this connection, when a lower alkyl ester is used as the reactive derivative, it is preferable to carry out the reaction from under room temperature to under heat reflux.

### (Production Method 2)

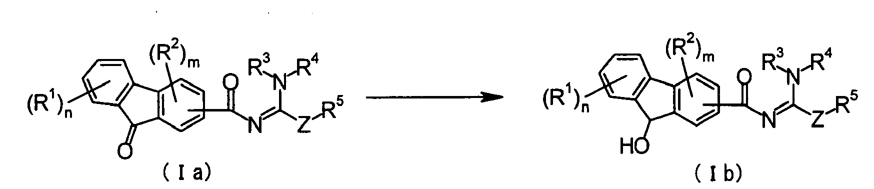

Among compounds (I), a compound (Ib) wherein -CR⁷R⁸- is represented by -CH(OH)- may be produced by subjecting a compound (Ia) of the invention in which said part is carbonyl group to a reduction reaction.
The reaction is carried out by treating the compound (Ia) with equivalent amount or an excess amount of a reducing agent. As the reducing agent, sodium borohydride, diisobutyl aluminum hydride or the like hydride reducing agent or a reducing agent described in "Comprehensive Organic Transformations" edited by Richard C. Larock (1989, VCH Publishers, Inc.) is used. The reaction is carried out using an aromatic hydrocarbon, an ether, DMF, DMSO, an alcohol (methanol, ethanol or the like) or water, or a mixture thereof, as the solvent, and performed under cooling to under heating, preferably at -20°C to room temperature.
When the compound (I) of the invention has various side chains and substituent groups, these compounds may be easily synthesized using the compound of the invention or a production intermediate thereof as the material, by using a method obvious to those skilled in the art or a modified method thereof. A such an example, a reaction in which substituent groups of the compounds obtained by the production methods 1 and 2 are further converted may be cited, and the reactions shown below may be employed.
A compound in which R⁷ and R⁸ in the formula (I) together represent =N-OR⁰ may be produced by carrying out dehydration condensation of the compound (Ia) of the invention wherein said moiety is oxo group and NH₂-OR⁰.
A compound in which one of R⁷ and R⁸ in the formula (I) is a halogen may be produced by subjecting the compound (Ib) of the invention wherein said moiety is -CH(OH)- to a halogenation reaction.
A compound in which R¹ in the formula (I) is -NH₂ may be produced by subjecting the compound of the invention wherein said moiety is -NO₂ to a reduction.

### (Production of starting compounds)

The starting compound (1) in the aforementioned production methods may be produced making use of conventionally known methods. 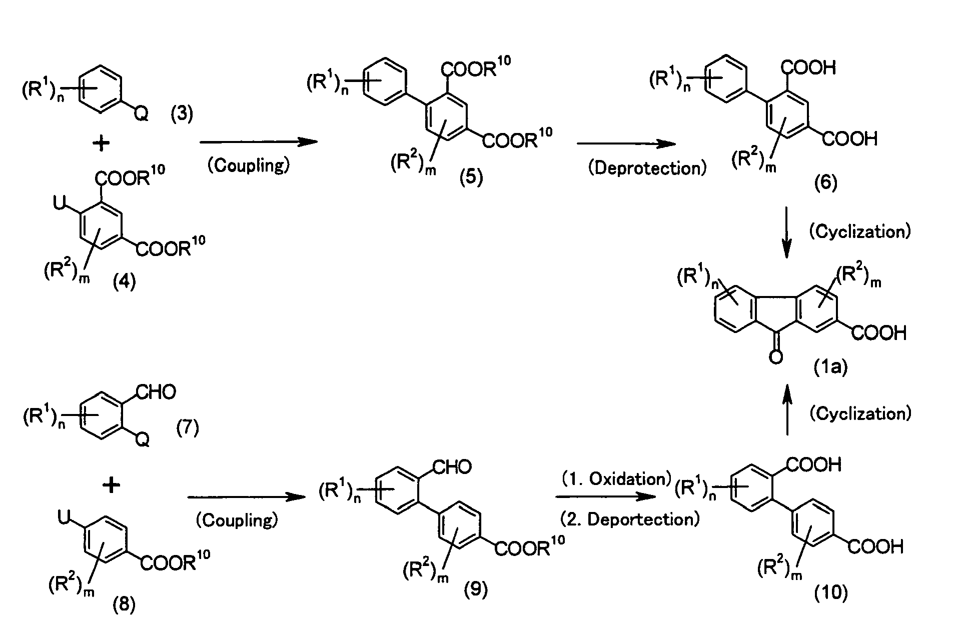 (In the reaction scheme, either one of Q and U is -Br, -Cl, -I or O-SO₂-CF₃, and the other is -B(OH)₂ or a B(O-lower alkyl)₂. R¹⁰ represents a lower alkyl or benzyl or the like protecting group.)
The compound (1a) in which R⁷ and R⁸ in the formula (1) together form oxo group and L¹ is hydroxyl group may be produced by the aforementioned reaction pathway.
In this reaction pathway, particularly the coupling reaction may be carried out by the method described in Synth. Commun., 11, 513 - 519 (1981), Synlett, 6, 829 - 831 (2000) or Chem. Lett., 1405 - 1408 (1989). The conventional intramolecular Friedel-Crafts' reaction may be used in the cyclization reaction, and the method described in J. Am. Chem. Soc., 63, 1948 (1941) may be exemplified. The oxidation reaction may be carried out at room temperature or under heating in DMF, methanol, water or the like solvent or in a mixture of them, using silver oxide, pyridinium dichromate, sodium chlorite or the like oxidizing agent.

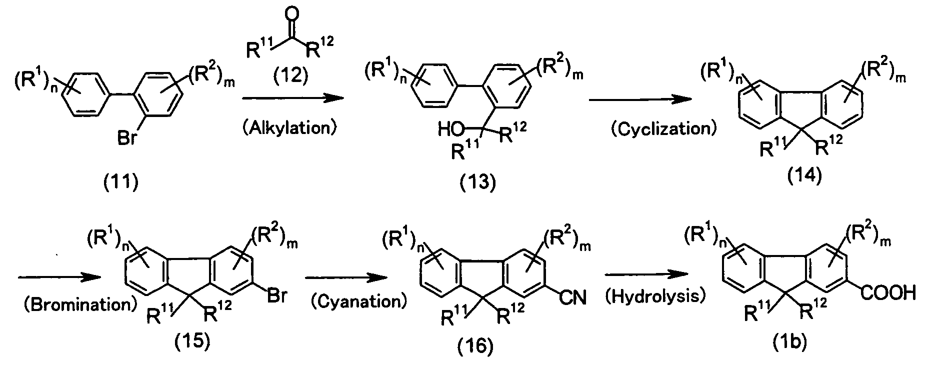

(In the reaction scheme, R¹¹ and R¹² represent a lower alkyl which may be substituted, or R¹¹ and R¹² together form a lower alkylene which may be interrupted by -O- or -NR⁶-.)
The compound (1b) in which at least one of R⁷ and R⁸ in the formula (1) is an alkyl group may be produced by bromination of the aromatic ring of the fluorene (14) which is obtained with reference to the method described in *J*. *Am. Chem. Soc.,* 63, 1948 (1941), making this into cyano group and then converting it into carboxyl group. In this case, the bromination may be carried out in accordance with the method described in "Orgnikum" edited by H. Becher, p. 189, 1973, and the cyanation with that in *J. Org. Chem.*, 26, 2522 (1961).

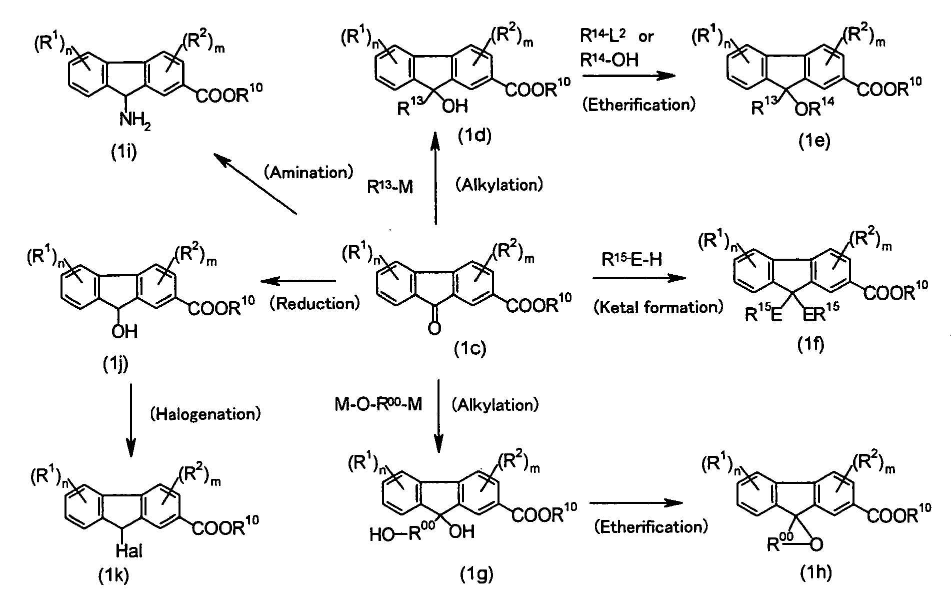

(In the reaction scheme, R¹³ and R¹⁴ represent a lower alkyl, R¹⁵ represents a lower alkyl or two R¹⁵ groups together represent a lower alkylene, M represents lithium ion, magnesium ion or the like counter cation of an organometalic reagent, E represents -O- or S-, L² represents halogen, -O-methanesulfonyl, O-p-toluenesulfonyl or the like leaving group, and Hal represents a halogen.)
Among the starting compounds (1), a compound in which at least one of R⁷ and R⁸ has various substituent groups may be easily produced from a 9-keto compound (1c) using each of the alkylation, etherification, ketal formation, amination, reduction and halogenation, or by a combination thereof.
Particularly, the alkylation may be carried out using Grignard reagent, organic lithium reagent, organic cerium reagent or the like organic metal reagent. The etherification for producing compound (1e) from (1d) is carried out using R¹⁴-L² as the alkylation agent, in the presence of sodium hydride, potassium hydride, potassium tert-butoxide, silver oxide or the like base. Alternatively, there is a case in which this alkylation is carried out under an acidic condition using R¹⁴-OH, and the reaction is carried out at room temperature to heating in methanol, ethanol, benzene, toluene, xylene or the like solvent, using toluenesulfonic acid or the like acid catalyst, iron nitrate or iron perchlorate or the like Lewis acid.
In the aforementioned reaction pathway, each of the alkylation, amination and reduction may also be carried out using the carboxyl compound (la) instead of the starting compound (1c). In addition, regarding each of the compounds (1c) to (1k), it may be converted into corresponding carboxyl compound through deprotection of the COOR¹⁰ group.

The compound (I) produced in this manner is isolated and purified directly as its free form or as a salt by subjecting it to a salt formation treatment in the usual way. The isolation and purification are carried out by employing general chemical operations such as extraction, concentration, evaporation, crystallization, filtration, recrystallization, various chromatographic treatments and the like.
Various isomers may be isolated in the usual way making use of a difference in physicochemical property between isomers. For example, optical isomers may be separated and purified by a method in which a racemic compound is made into a diastereomeric mixture of its salts with an optically active organic acid (tartaric acid or the like) and then subjected to fractional recrystallization, or a column chromatography packed with chiral stationary phase or the like technique. In addition, an optically active compound may also be produced using an appropriate optically active compound as the material. In this connection, a mixture of diastereomers may also be separated by fractional crystallization, chromatography and the like.

According to the "combination product for treating IBS" of the invention, the "combination product" means pharmaceutical preparations having independent components, which can be used in a concomitant therapy, and which may also be put on the market by packaging them in combination (e.g., a kit or the like form) or each independently for use in a concomitant administration. In this case, the "simultaneously" means that the first pharmaceutical preparation and the second pharmaceutical preparation are administered together, and the "separately" means that the first pharmaceutical preparation and the second pharmaceutical preparation are separately administered through the same or different route of administration at the same or different administration frequency or administration interval. Preferably, by taking bioavailability, safety and the like of respective pharmaceutical preparations into consideration, they are administered simultaneously or separately under the preparation formulation, route of administration, administration frequency and the like conditions suited for respective pharmaceutical preparations. When durations of the active ingredients of the first pharmaceutical preparation and the second pharmaceutical preparation are almost the same, it is desirable that these are administered simultaneously or within 1 hour in order. When simultaneously administered, the separately prepared preparations may be administered by mixing them using a diluent or the like prior to use.
An example of the kit is a package which contains a first pharmaceutical preparation comprising a 5-HT_{2B} selective antagonistic compound and a second pharmaceutical preparation comprising a 5-HT₇ selective antagonistic compound, and, as occasion demands, may also contain a placebo or the like additional pharmaceutical preparation and a display member, that facilitate their administration at their respective administration times.

The medicaments of the invention for IBS, which comprises a selective dual antagonist for 5-HT_{2B} and 5-HT₇ receptors as the active ingredient, and the aforementioned first pharmaceutical preparation or second pharmaceutical preparation constituting the combination product of the invention, can be prepared by generally used methods using medicinal carriers, fillers and the like which are generally used in said field. The administration may be either oral administration by tablets, pills, capsules, granules, powders, solutions and the like, or parenteral administration by injections for intravenous injection, intramuscular injection and the like, ointments, plaster preparations, creams, jellies, cataplasmas, sprays, lotions, eye drops, eye ointments and the like external preparations, suppositories, inhalations and the like.
As the solid composition for oral administration by the invention, tablets, powders, granules and the like are used. In such a solid composition, one or more active substances are mixed with at least one inert filler such as lactose, mannitol, glucose, hydroxypropylcellulose, microcrystalline cellulose, starch, polyvinyl pyrrolidone, aluminum magnesium silicate or the like. In accordance with the usual way, the composition may contain inert additives such as magnesium stearate and the like lubricants, carboxymethylstarch sodium and the like disintegrators and solubilizing agents. As occasion demands, the tablets or pills may be coated with a sugar coating or a gastric or enteric coating.

As the liquid composition for oral administration, pharmaceutically acceptable emulsions, solutions, suspensions, syrups, elixirs and the like are included, and generally used inert solvents such as purified water, ethanol and the like can be used.
In addition to the inert solvents, this composition may contain solubilizing agents, moistening agents, suspending agents and the like auxiliary agents, sweeteners, correctives, aromatics and antiseptics.
As the injections for parenteral administration, sterile aqueous or non-aqueous solutions, suspensions and emulsions are included. As the aqueous solvent, for example, distilled water for injection and physiological saline are included. Examples of the non-aqueous solvent include propylene glycol, polyethylene glycol, olive oil or the like plant oil, ethanol or the like alcohols, polysorbate 80 (name in the Pharmacopeia) and the like. Such a composition may further contain tonicity agents, antiseptics, moistening agents, emulsifying agents, dispersing agents, stabilizing agents and solubilizing agents. These are sterilized by, for example, filtration through a bacteria retaining filter, formulation of bactericides or irradiation. In addition, these can also be used by producing a sterile solid compositions and dissolving or suspending them in sterile water or a sterile solvent for injection prior to use.

When the medicament for IBS of the invention is an antagonist which comprises the "5-HT_{2B} selective antagonistic compound" as the first component and the "5-HT₇ selective antagonistic compound" as the second component, blending amounts of the respective compounds are optionally decided in response to the symptom of individual patient, within the clinically effective amounts in the case of the prescription as respective single preparations.
In addition, when the medicament for IBS of the invention is an antagonist which comprises a selective dual antagonistic compound for 5-HT_{2B} and 5-HT₇, its daily dose per body weight in the case of oral administration is generally from about 0.001 to 50 mg/kg, preferably from 0.01 to 30 mg/kg, further preferably from 0.05 to 10 mg/kg, and its daily dose per body weight in the case of intravenous administration is generally from about 0.0001 to 10 mg/kg, preferably from 0.001 to 1.0 mg/kg, and this is administered once a day or by dividing the daily dose into two or more doses. The dose is optionally decided in response to individual case, by taking symptom, age, sex and the like into consideration.
Dose of the compounds as respective active ingredients of the aforementioned first pharmaceutical preparation and second pharmaceutical preparation which constitute the combination product of the invention is optionally decided in response to the symptom of individual patient, within the clinically effective amounts in the case of the prescription as respective single preparations.

### Production Examples

The following describes concrete preparations of the compounds described in formula (I) which is active ingredients in the drugs of the invention. In this connection, production methods of starting compounds are described as Reference Production Examples.

### Reference Production Example 1-a

Diethyl 2'-methylbiphenyl-2,4-dicarboxylate was obtained by allowing 4-bromoisophthalic acid diethyl ester to react with 2-methylphenylboronic acid, sodium carbonate and tetrakistriphenylphosphine palladium under heating in toluene-ethanol-water. FAB-MS: 313 (M + H)⁺.

### Reference Production Example 1-b

2'-Methylbiphenyl-2,4-dicarboxylic acid was obtained by treating ethanol solution of diethyl 2'-methylbiphenyl-2,4-dicarboxylate with 1 M sodium hydroxide. FAB-MS: 257 (M + H)⁺.

### Reference Production Example 1-c

5-Methyl-9-oxo-9H-fluorene-2-carboxylic acid was obtained by heating 2'-methylbiphenyl-2,4-dicarboxylic acid in polyphosphoric acid. FAB-MS: 239 (M + H)⁺.

### Reference Production Example 2

3'-Methylbiphenyl-2,4-dicarboxylic acid was treated in the same manner as in Reference Production Example 1-c, and then the thus obtained solid was heated in ethanol in the presence of concentrated sulfuric acid to carry out esterification. After treatment of the reaction, this was separated and purified by a silica gel column chromatography to obtain ethyl 6-methyl-9-oxo-9H-fluorene-2-carboxylate [FAB-MS: 267 (M + H)⁺] and ethyl 8-methyl-9-oxo-9H-fluorene-2-carboxylate [FAB-MS: 267 (M + H)⁺], respectively.

### Reference Production Example 3

In the same manner as in Reference Production Example 1-a, ethyl 3-chloro-2'-formylbiphenyl-4-carboxylate was produced from ethyl 4-bromo-2-chlorobenzoate and 2-formylphenylboronic acid. FAB-MS: 288 (M)⁺.
3'-Chloro-4'-(ethoxycarbonyl)biphenyl-2-carboxylic acid was obtained by allowing ethyl 3-chloro-2'-formylbiphenyl-4-carboxylate to react with sodium perchlorate, sodium dihydrogenphosphate and 2-methyl-2-butene in tert-butanol-acetonitrile-water at room temperature. FAB-MS: 305 (M + H)⁺.
Thereafter, in the same manner as in Reference Production Example 1-c and Reference Production Example 2, ethyl 1-chloro-9-oxo-9H-fluorene-2-carboxylate [FAB-MS: 287 (M + H)⁺] and ethyl 3-chloro-9-oxo-9H-fluorene-2-carboxylate [FAB-MS: 287 (M + H)⁺] were produced, respectively.

### Reference Production Example 4-a

9-Hydroxy-9-methyl-9H-fluorene-2-carboxylic acid was obtained by allowing 9-oxo-9H-fluorene-2-carboxylic acid to react with methyl lithium in THF at from -20°C to 0°C. FAB-MS: 239 (M - H)⁻.

### Reference Production Example 4-b

Methyl 9-hydroxy-9-methyl-9H-fluorene-2-carboxylate was obtained by allowing 9-hydroxy-9-methyl-9H-fluorene-2-carboxylic acid to react with sodium bicarbonate and methyl iodide in DMF at room temperature. FAB-MS: 255 (M + H)⁺.

### Reference Production Example 4-c

Methyl 9-methoxy-9-methyl-9H-fluorene-2-carboxylate was obtained by allowing methyl 9-hydroxy-9-methyl-9H-fluorene-2-carboxylate to react with iron nitrate in methanol under heating. FAB-MS: 269 (M + H)⁺.

### Reference Production Example 5

Methyl 9-methoxymethyl-9-methyl-9H-fluorene-2-carboxylate was obtained by allowing methyl 9-hydroxy-9-methyl-9H-fluorene-2-carboxylate to react with sodium hydride and methoxymethyl chloride in DMF at room temperature. FAB-MS: 298 (M)⁺.

### Reference Production Example 6-a

In THF, methylmagnesium bromide was allowed to act upon 3-chloropropan-1-ol to form magnesium oxide, and then magnesium metal was allowed to react therewith, thereby preparing a Grignard reagent (ClMg(CH₂)₃OMgBr). This was allowed to react with 9-oxo-9H-fluorene-2-carboxylic acid in the same manner as in Reference Production Example 4-a, and then the thus obtained 9-hydroxy-9-hydroxypropyl-9H-fluorene-2-carboxylic acid was allowed to react with methyl iodide in the same manner as in Reference Production Example 4-b, thereby obtaining methyl 9-hydroxy-9-hydroxypropyl-9H-fluorene-2-carboxylate. FAB-MS: 297 (M - H)⁻.

### Reference Production Example 6-b

Methyl 4',5'-dihydro-3'H-spiro[fluorene-9,2'-furan]-2-carboxylate was obtained by allowing methyl 9-hydroxy-9-hydroxypropyl-9H-fluorene-2-carboxylate to undergo the reaction under heating in toluene in the presence of p-toluenesulfonic acid.
FAB-MS: 281 (M + H)⁺.

### Reference Production Example 7-a

Methyl 8-bromomethyl-9-oxo-9H-fluorene-2-carboxylate was obtained by allowing methyl 8-methyl-9-oxo-9H-fluorene-2-carboxylate to react with N-bromosuccinimide and 2,2'-azobisisobutyronitrile under heating in carbon tetrachloride.
EI-MS: 330 (M)⁺, 332 (M + 2)⁺.

### Reference Production Example 7-b

Methyl 8-dimethylaminomethyl-9-oxo-9H-fluorene-2-carboxylate was obtained by allowing methyl 8-bromomethyl-9-oxo-9H-fluorene-2-carboxylate to react with dimethylamine (2 M, methanol solution) and potassium carbonate at room temperature in THF. FAB-MS: 296 (M + H)⁺.

### Reference Production Example 8-a

Methyl 8-acetoxymethyl-9-oxo-9H-fluorene-2-carboxylate was obtained by allowing methyl 8-bromomethyl-9-oxo-9H-fluorene-2-carboxylate to react with potassium acetate at room temperature in DMF. FAB-MS: 311 (M + H)⁺.

### Reference Production Example 8-b

Methyl 8-hydroxymethyl-9-oxo-9H-fluorene-2-carboxylate was obtained by allowing methyl 8-acetoxymethyl-9-oxo-9H-fluorene-2-carboxylate to react with potassium carbonate at room temperature in methanol-THF. FAB-MS: 269 (M + H)⁺.

### Reference Production Example 8-c

Methyl 8-methoxymethyl-9-oxo-9H-fluorene-2-carboxylate was obtained by allowing methyl 8-hydroxymethyl-9-oxo-9H-fluorene-2-carboxylate to react with methyl iodide and silver oxide under heating in acetonitrile. FAB-MS: 283 (M + H)⁺.

### Reference Production Example 9

Methyl 9-fluoro-9H-fluorene-2-carboxylate was obtained by allowing methyl 9-oxo-9H-fluorene-2-carboxylate to react with sodium borohydride at room temperature in methanol to reduce the carbonyl group, and then allowing the thus obtained compound to react with diethylaminosulfur trifluoride at room temperature in methylene chloride. FAB-MS: 243 (M + H)⁺.

### Reference Production Example 10

Propyl spiro[1,3-dioxolane-2,9'-fluorene]-2'-carboxylate was obtained by allowing propyl 9-oxo-9H-fluorene-2-carboxylate to react with ethylene glycol and p-toluenesulfonic acid under heating in benzene. FAB-MS: 311 (M + H)⁺.

### Reference Production Example 11

Propyl 9,9-dimethoxy-9H-fluorene-2-carboxylate was obtained by allowing propyl 9-oxo-9H-fluorene-2-carboxylate to react with methyl orthoformate and acetyl chloride in methanol at room temperature. FAB-MS: 313 (M + H)⁺.

### Reference Production Example 12

5'-Fluorospiro[1,3-dithiolane-2,9'-fluorene]-2'-carboxylate was obtained by allowing 5-fluoro-9-oxo-9H-fluorene-2-carboxylate to react with 1,2-ethanedithiol and boron trifluoride diethyl ether complex under heating in acetic acid. ESI-MS: 317 (M - H)⁻.

### Reference Production Example 13-a

Methyl (9EZ)-9-hydroxyimino-9H-fluorene-2-carboxylate was obtained by allowing methyl 9-oxo-9H-fluorene-2-carboxylate to react with hydroxylamine hydrochloride at room temperature in pyridine. FAB-MS: 254 (M + H)⁺.

### Reference Production Example 13-b

Methyl 9-acetylamino-9H-fluorene-2-carboxylate was obtained by treating methyl (9EZ)-9-hydroxyimino-9H-fluorene-2-carboxylate with 10% palladium-carbon and acetic anhydride in dioxane in an atmosphere of hydrogen gas. FAB-MS: 282 (M + H)⁺.

### Reference Production Example 14-a

1-Biphenyl-2-ylcyclopentanol was obtained by allowing 2-bromobiphenyl to react with n-butyl lithium (1.58 M, hexane solution) at -78°C in THF, and then adding THF solution of cyclopentanone and carrying out the reaction at room temperature.
EI-MS: 238 (M)⁺.

### Reference Production Example 14-b

Spiro[cyclopentane-1,9'-fluorene] was obtained by allowing 1-biphenyl-2-ylcyclopentanol to undergo the reaction under heating in formic acid. EI-MS: 220 (M)⁺.

### Reference Production Example 14-c

2'-Bromospiro[cyclopentane-1,9'-fluorene] was obtained by bromination described in J. Am. Chem. Soc., 80, 4327 (1958) using spiro[cyclopentane-1,9'-fluorene]. EI-MS: 298 (M)⁺, 300 (M + 2)⁺.

### Reference Production Example 14-d

Spiro[cyclopentane-1,9'-fluorene]-2'-carbonitrile was obtained by allowing 2'-bromospiro[cyclopentane-1,9'-fluorene] to react with cupper cyanide under heating in DMF. ESI-MS: 246 (M + H)⁺.

### Reference Production Example 14-e

Spiro[cyclopentane-1,9'-fluorene]-2'-carboxylic acid was obtained by allowing ethanol solution of spiro[cyclopentane-1,9'-fluorene]-carbonitrile to react with 8 M potassium hydroxide aqueous solution under heating. ESI-MS: 263 (M + H)⁺.

### Reference Production Example 15

4-(4'-Methylbiphenyl2-yl)tetrahydro-2H-pyran-4-ol [EI-MS: 268 (M)⁺] was obtained by carrying out the reaction of Reference Production Example 14-a using 2-bromo-4'-methylbiphenyl and tetrahydro-4H-pyran-4-one as the starting materials, and then 2-methyl-2',3',5',6'-tetrahydrospiro[fluorene-9,4'-pyran] [ESI-MS: 251 (M + H)⁺] was produced in the same manner as in Reference Production Example 14-b. By allowing this to react with potassium permanganate under heating in pyridine-water, and allowing the thus obtained crude product to undergo the reaction under heating in methanol in the presence of sulfuric acid, methyl-2',3',5',6'-tetrahydrospiro[fluorene-9,4'-pyran]-2'-carboxylate was obtained. ESI-MS: 295 (M + H)⁺.

### Reference Production Example 16-a

9H-Fluorene-9,9-diyldimethylene dimethanesulfonate was obtained by allowing 9H-fluorene-9,9-diyldimethanol to react with methanesulfonyl chloride and triethylamine at room temperature in methylene chloride. EI-MS: 382 (M)⁺.

### Reference Production Example 16-b

9,9,-Bis(iodomethyl)-9H-fluorene was obtained by allowing 9H-fluorene-9,9-diyldimethylene dimethanesulfonate to react with sodium iodide under heating in hexamethylphosphoramide. By treating this with zinc under heating in ethanol, spiro[cyclopropane-1,9'-fluorene] was obtained (EI-MS: 192 (M)⁺). Thereafter, spiro[cyclopropane-1,9'-fluorene]-2'-carboxylic acid was produced in the same manner as in Reference Production Examples 14-c to 14-e. ESI-MS: 235 (M - H)⁻.

### Reference Production Example 17-a

9,9-Bis[2-(benzyloxy)ethyl]-2-bromo-9H-fluorene was obtained by allowing 2-bromo-9H-fluorene and [(2-chloroethoxy)methyl]benzene to undergo the reaction under heating in DMSO in the presence of potassium tert-butoxide. FAB-MS: 535 (M + Na)⁺, 537 (M + 2 + Na)⁺.

### Reference Production Example 17-b

Ethyl 9,9-bis[2-(benzyloxy)ethyl]-9H-fluorene-2-carboxylate was obtained by cyanation of the bromo group of 9,9-bis[2-(benzyloxy)ethyl]-2-bromo-9H-fluorene in the same manner as in Reference Production Example 14-d and subsequently converting into carboxyl group by the same hydrolysis reaction of Reference Production Example 14-e, and then carrying out esterification reaction in the same manner as in Reference Production Example 4-b using ethyl iodide. FAB-MS: 507 (M + H)⁺.

### Reference Production Example 17-c

Ethyl 9,9-bis(2-hydroxyethyl)-9H-fluorene-2-carboxylate was obtained by allowing ethyl 9,9-bis[2-(benzyloxy)ethyl]-9H-fluorene-2-carboxylate to react with palladium-carbon at room temperature in methanol in an atmosphere of hydrogen gas (FAB-MS: 327 (M + H)⁺). The thus obtained compound was allowed to react with p-toluenesulfonyl chloride at room temperature in methylene chloride in the presence of triethylamine, and then the thus obtained compound was allowed to react with methylamine (40% methanol solution) under heating in dioxane in the presence of potassium carbonate to obtain methyl 1'-methylspiro[fluorene-9,4'-piperidine]-2-carboxylate. APCI: 308 (M + H)⁺.

### Reference Production Example 18-a

Methyl 9,9-bis(hydroxymethyl)-9H-fluorene-2-carboxylate was obtained by allowing methyl 9H-fluorene-2-carboxylate to react with paraformaldehyde at room temperature in DMSO in the presence of sodium ethoxide. FAB-MS: 284 (M)⁺.

### Reference Production Example 18-b

Methyl 9,9-bis({[tert-butyl(dimethyl)silyl]oxy} methyl)-9H-fluorene-2-carboxylate was obtained by allowing methyl 9,9-bis(hydroxymethyl)-9H-fluorene-2-carboxylate to react with tert-butyldimethylsilyl chloride at room temperature in pyridine. FAB-MS: 513 (M + H)⁺.

### Reference Production Example 19

9-Hydroxy-9-(tetrahydro-2H-pyran-4-yl)-9H-fluorene-2-carboxylic acid [FAB-MS: 309 (M - H)⁻] produced from 4-tetrahydro-2H-pyranyl magnesium chloride (prepared from 4-chlorotetrahydro-2H-pyran and magnesium) and 9-oxo-9H-fluorene-2-carboxylic acid in the same manner as in Reference Production Example 4-a was allowed to react with triethylsilane at room temperature in trifluoroacetic acid, thereby obtaining 9-(tetrahydro-2H-pyran-4-yl)-9H-fluorene-2-carboxylic acid. FAB-MS: 291 (M - H)⁻.

### Reference Production Example 20

9-(Tetrahydro-4H-pyran-4-ylidene)-9H-fluorene-2-carboxylic acid was obtained by allowing 9-hydroxy-9-(tetrahydro-2H-pyran-4-yl)-9H-fluorene-2-carboxylic acid to react with 6 M hydrochloric acid under heating in dioxane. FAB-MS: 294 (M)⁺.

### Reference Production Example 21-a

2-Fluoro-4'-methyl-6-nitrobiphenyl [FAB-MS: 232 (M + H)⁺] was obtained from 2-fluoro-6-nitrophenyl trifluoromethanesulfonate and 4-methylphenylboric acid by carrying out the reaction in the same manner as in Reference Production Example 1-a, and converted into (6-fluoro-4'-methylbiphenyl-2-yl)amine [EI-MS: 201 (M)⁺] by subjecting this nitro group to catalytic hydrogenation reduction, and then Sandmeyer reaction was carried out to obtain 2-bromo-6-fluoro-4'-methylbiphenyl. EI-MS: 266 (M)⁺, 268 (M+2)⁺.

### Reference Production Example 21-b

4-(6-Fluoro-4'-methylbiphenyl-2-yl)tetrahydro-2H-pyran-4-ol [EI-MS: 286 (M)⁺] was obtained by carrying out the reaction of 2-bromo-6-fluoro-4'-methylbiphenyl with tetrahydro-4H-pyran-4-one in the same manner as in Reference Production Example 14-a using t-butyl lithium (1.48 M, pentane solution), and then further reaction was carried out in the same manner as in Reference Production Example 14-b to obtain 5-fluoro-2-methyl-2',3',5',6'-tetrahydrospiro[fluorene-9,4'-pyran]. FAB-MS:268 (M)⁺.

### Reference Production Example 21-c

5-Fluoro-2',3',5',6'-tetrahydrospiro[fluorene-9,4'-pyran]-2-carboaldehyde [FAB-MS: 283 (M + H)⁺] was obtained by allowing 5-fluoro-2-methyl-2',3',5',6'-tetrahydrospiro[fluorene-9,4'-pyran], N-bromosuccinimide and 2,2'-azobisisobutyronitrile to undergo the reaction under heating in carbon tetrachloride, and by allowing the thus obtained crude product to react with silver nitrate in acetone-water. By further allowing this compound to react with sodium perchlorate, sodium dihydrogenphosphate and 2-methyl-2-butene at room temperature in a mixed solvent of tert-butanol-acetonitrile-water, 5-fluoro-2',3',5',6'-tetrahydrospiro[fluorene-9,4'-pyran]-2-carboxylic acid was obtained. FAB-MS: 299 (M + H)⁺.
Compounds of Reference Production Examples 22 to 111 were produced in the same manner as in the above Reference Production Examples. Their structural formulae and physical properties are shown in the Tables 1 to 6 which are described later.

### Production Example 1

A 402 mg portion of CDI was added to 20 ml DMF solution of 400 mg 5-fluoro-9-oxo-9H-fluorene-2-carboxylic acid and stirred at 50°C for 1 hour. After cooling to room temperature, 743 mg of guanidine carbonate was added thereto and stirred overnight. After evaporation of the solvent, water was added thereto, and the thus precipitated solid was purified by a silica gel column chromatography (Chromatorex (registered trademark), methanol/chloroform) to obtain 434 mg of N-(diaminomethylene)-5-fluoro-9-oxo-9H-fluorene-2-carboxamide as yellow solid.

### Production Example 2

A 2.67 g portion of 1,1'-carbonyldiimidazole was added to 60 ml dimethylformamide (DMF) solution of 3.35 g 9-oxo-9H-fluorene-2-carboxylic acid and stirred at room temperature for 2.25 hours. This solution was added under ice-cooling to a solution which had been prepared by adding 3.00 g of sodium hydride to 20 ml DMF solution of 7.16 g guanidine hydrochloride and stirring at room temperature for 1.5 hours, and the mixture was stirred at room temperature for 1.5 hours. After evaporation of the solvent, water and ethyl acetate were added thereto, and the precipitated solid was washed with methanol to obtain 3.00 g ofN-(diaminomethylene)-9-oxo-9H-fluorene-2-carboxamide as yellow solid.

### Production Example 3

A 110 mg portion of sodium borohydride was added to 10 ml methanol solution of 400 mg N-(diaminomethylene)-9-oxo-9H-fluorene-2-carboxamide and stirred at room temperature for 1 hour. After evaporation of the solvent, chloroform and 1 M sodium hydroxide aqueous solution were added thereto, and the precipitated solid was dissolved in 30 ml of ethanol, mixed with 0.2 ml of 4 M hydrogen chloride-ethyl acetate solution and stirred at room temperature for 1.5 hours. By collecting the thus formed solid by filtration, 380 mg ofN-(diaminomethylene)-9-hydroxy-9H-fluorene-2-carboxamide hydrochloride was obtained as white solid.

### Production Example 4

A 1.0 g portion of sulfonyl chloride was added to 20 ml methylene chloride suspension of 480 mg N-(diaminomethylene)-9-hydroxy-9H-fluorene-2-carboxamide and stirred at room temperature for 30 minutes. After evaporation of the solvent, the residue was purified by a column chromatography (silica gel 60, methanol/chloroform) to obtain 155 mg of 9-chloro-N-(diaminomethylene)-9H-fluorene-2-carboxamide.

### Production Example 5

A 2 ml portion of 4 M hydrogen chloride-ethyl acetate solution was added to 10 ml methanol solution of 170 mg of tert-butyl (2-{[(diaminomethylene)amino]carbonyl}-9H-fluoren-9-yl)carbamate produced in the same manner as in Production Example 1, and the mixture was stirred at 60°C for 20 minutes. By washing the thus obtained solid with hot ethanol, 83 mg of 9-amino-N-(diaminomethylene)-9H-fluorene-2-carboxamide dihydrochloride was obtained.

### Production Example 6

A 1 ml portion of 4 M hydrogen chloride-methanol solution was added to 5 ml methanol solution of 490 mg N-(diaminomethylene)-9,9-bis({[tert-butyl(dimethyl)silyl]oxy}methyl)-9H-fluorene-2-carboxamide and stirred at room temperature for 2 hours. By evaporating the solvent and washing the residue with ethyl acetate, 250 mg of N-(diaminomethylene)-9,9-bis(hydroxymethyl)-9H-fluorene-2-carboxamide hydrochloride was obtained.

### Production Example 7

A 20 mg portion of hydroxylamine hydrochloride was added to 3 ml pyridine solution of 55 mg N-(diaminomethylene)-8-hydroxymethyl-9-oxo-9H-fluorene-2-carboxamide and stirred at room temperature for 10 hours. After evaporation of the solvent, water-ethanol was added thereto, and the precipitated solid was purified by Chromatorex (methanol/chloroform) to obtain 14 mg of (9EZ)-N-(diaminomethylene)-9-hydroxyimino-8-hydroxymethyl-9H-fluorene-2-carboxamide as white solid.

### Production Example 8

A 420 mg portion of (2R)-2-[(tert-butoxycarbonyl)amino]-3-methylbutanoic acid, 400 mg of 1-[3-(dimethylamino)propyl]-3-ethylcarbodiimide and 22 mg of 4-(N,N-dimethylamino)pyridine were added to 8 ml DMF solution of 470 mg N-(diaminomethylene)-9-hydroxy-9H-fluorene-2-carboxamide and stirred overnight at room temperature. After evaporation of the solvent, chloroform and saturated sodium bicarbonate aqueous solution were added thereto to remove the insoluble matter, and the organic layer was washed with saturated brine and dried with magnesium sulfate. After evaporation of the solvent, the residue was purified by a column chromatography (silica gel 60, methanol/chloroform). A 270 mg portion of the thus obtained compound was dissolved in 10 ml of ethanol, mixed with 2 ml of 4 M hydrogen chloride-ethyl acetate solution and stirred overnight at 40°C. After evaporation of the solvent, the residue was recrystallized from 2-propanol to obtain 30 mg of 9-hydroxy-N-[(2EZ,4S)-4-isopropyl-S-oxoimidazolin-2-ylidene-9H-fluorene-2-carboxamide hydrochloride.

### Production Example 9

A 2.99 ml portion of sodium methoxide-methanol solution (28%) was added to 8 ml DMF solution of 1.38 g guanidine hydrochloride and stirred at room temperature for 1 hour. A 4 ml portion of DMF solution of 350 mg ethyl 6-methyl-9-oxo-9H-fluorene-2-carboxylate was added to this solution and stirred at 100°C for 3 hours. This was spontaneously cooled to room temperature, the solvent was evaporated, and then methanol, water and 1 M sodium hydroxide aqueous solution were added thereto, and the thus precipitated solid was washed with 5 ml of methanol to obtain 215 mg of N-(diaminomethylene)-6-methyl-9-oxo-9H-fluorene-2-carboxamide as yellow solid.

Compounds of Production Examples 10 to 110 were produced in the same manner as in the above Production Examples. Their structural formulae and physical properties are shown in the Tables 7 to 18 which are described later. In addition, the compounds of Table 19 and Table 20, which are described later, may be easily produced in almost the same manner as in the above Production Examples or the methods described in the production methods, or by applying slight modifications obvious for those skilled in the art to these methods.
In this connection, the 6 compounds of Production Examples 56a and 56b, 60a and 60b, 78a and 78b were produced by carrying out resolution of respective compounds of Production Examples 56, 60 and 77 produced as racemic compounds, using chiral columns. The used columns and solvents used as the mobile phase are shown below.

### Production Examples 56a and 56b

Used column: CHIRALPAK AD-H, mobile phase: methanol/diethylamine.

### Production Examples 60a and 60b

Used column: CHIRALPAK OJ, mobile phase: ethanol/diethylamine.

### Production Examples 78a and 78b

Used column: CHIRALPAK AD-H, mobile phase: hexane/ethanol/triethylamine.

### Production Examples 111

2-amino-4-(4-fluoronaphtho-1-yl)-6-isopropylpyrimidine(hereinafter abbreviated as RS-127445) was prepared in the same manner as described in the international application WO97/44326, and (R)-3-(2-(2-(4-methylpiperidine-1-yl)ethyl)pyrrolidine-1-sulfonyl)phenol, (hereinafter abbreviated as SB-269970) was prepared in the same manner as described in the international application WO97/48681, respectively.

Symbols in the tables have the following meanings. Each numeral before the substituent group shows the substituted position.
Me, methyl, Et: ethyl, Bu: normal butyl, REx: Reference Production Example number, Ex: Production Example number, Cmp: compound number, RSyn and Syn: production methods (numerals indicate Reference Production Example number and Production Example number of compound produced in the same manner), Str: structure, Sal: salt (not described: free form; HCl: hydrochloride; numeral indicates molar ratio of the acid component, for example, 2 HCl means dihydrochloride), Dat: physicochemical properties(FAB: FAB-MS, ESI: ESI-MS, EI: EI-MS, NMR: δ values of typical peaks of nuclear magnetic resonance spectrum (DMSO-_{d6}, TMS internal standard)).
In this connection, the compound in which "*" was added to the substituent group in respective table indicates that it is one of the chiral compounds obtained by separation of optical isomers based on the asymmetry of carbon bonded to said substituent group. In addition, the following symbols shown in the tables show analytical conditions of high performance liquid chromatography. Proc. A: (column: CHIRALPAK AD-H [0.46 cm I.D. x 25 cm], mobile phase, methanol/diethylamine = 100/0.1, flow rate: 0.5 ml/min, temperature: 20°C, wavelength: 260 nM), Proc. B: (column: CHIRALPAK OJ [0.46 cm I.D. x 25 cm], mobile phase, ethanol/diethylamine = 100/0.1, flow rate: 0.3 ml/min, temperature: 40°C, wavelength: 264 nM), Proc. C: (column: CHIRALPAK AD-H [0.46 cm I.D. x 25 cm], mobile phase, hexane/ethanol/triethylamine = 50/50/0.05, flow rate: 1.0 ml/min, temperature: 25°C, wavelength: 257 nM).

**Table 1**

| | | | | |
|---|---|---|---|---|
| 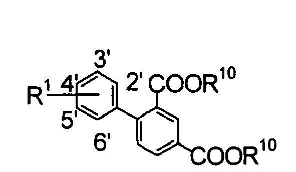 | | | | |

| REx | RSyn | R¹ | R¹⁰ | Dat |
|---|---|---|---|---|
| 22 | 1-a | 2'-F | -Et | ESI : 317 (M+H)⁺ |
| 23 | 1-a | 3'-F | -Et | FAB : 317 (M+H)⁺ |
| 24 | 1-a | 4'-F | -Et | FAB : 317 (M+H)⁺ |
| 25 | 1-a | 2'-Cl | -Et | FAB : 333 (M+H)⁺ |
| 26 | 1-a | 3'-Cl | -Et | FAB : 333 (M+H)⁺ |
| 27 | 1-a | 4'-Cl | -Et | FAB : 333 (M+H)⁺ |
| 28 | 1-a | 3'-Me | -Et | FAB : 313 (M+H)⁺ |
| 29 | 1-a | 3'-OMe | -Et | FAB : 328 (M)⁺ |
| 30 | 1-a | 4'-OMe | -Et | FAB : 329 (M+H)⁺ |
| 31 | 1-a | 2'-Et | -Et | ESI : 327 (M+H)⁺ |
| 32 | 1-a | 2'-CF₃ | -Et | FAB : 367 (M+H)⁺ |
| 33 | 1-a | 2'-F, 5'-Me | -Et | ESI : 331 (M+H)⁺ |
| 34 | 1-a | 2'-Me, 5'-Me | -Et | ESI : 327 (M+H)⁺ |
| 35 | 1-b | 2'-F | -H | FAB : 261 (M+H)⁺ |
| 36 | 1-b | 3'-F | -H | FAB : 259 (M-H)⁻ |
| 37 | 1-b | 4'-F | -H | FAB : 261 (M+H)⁺ |
| 38 | 1-b | 2'-Cl | -H | FAB : 277 (M+H)⁺ |
| 39 | 1-b | 3'-Cl | -H | FAB : 277 (M+H)⁺ |
| 40 | 1-b | 4'-Cl | -H | FAB : 277 (M+H)⁺ |
| 41 | 1-b | 3'-Me | -H | FAB : 257 (M+H)⁺ |
| 42 | 1-b | 3'-OMe | -H | FAB : 271 (M-H)⁻ |
| 43 | 1-b | 4'-OMe | -H | FAB : 273 (M+H)⁺ |
| 44 | 1-b | 2'-Et | -H | FAB : 269 (M-H)⁻ |
| 45 | 1-b | 2'-CF₃ | -H | FAB : 309 (M-H)⁻ |
| 46 | 1-b | 2'-F, 5'-Me | -H | ESI : 273 (M-H)⁻ |
| 47 | 1-b | 2'-Me, 5'-Me | -H | FAB : 269 (M-H)⁻ |

**Table 2**

| | | | | | |
|---|---|---|---|---|---|
| 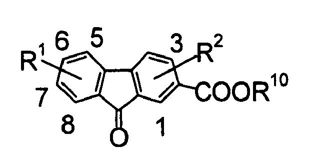 | | | | | |

| REx | RSyn | R¹ | R² | R¹⁰ | Dat |
|---|---|---|---|---|---|
| 48 | 1-c | 5-F | -H | -H | FAB : 242 (M)⁻ |
| 49 | 1-c | 7-F | -H | -H | FAB : 243 (M+H)⁺ |
| 50 | 1-c | 8-F | -H | -H | FAB : 243 (M+H)⁺ |
| 51 | 1-c | 5-Cl | -H | -H | FAB : 259 (M+H)⁺ |
| 52 | 1-c | 7-Cl | -H | -H | FAB : 259 (M+H)⁺ |
| 53 | 1-c | 8-Me | -H | -H | FAB : 239 (M+H)⁺ |
| 54 | 1-c | 5-Et | -H | -H | FAB : 251 (M-H)⁻ |
| 55 | 1-c | 5-CF₃ | -H | -H | FAB : 291 (M-H)⁻ |
| 56 | 1-c | 7-OMe | -H | -H | FAB : 255 (M+H)⁺ |
| 57 | 1-c | 5-F, 8-Me | -H | -H | ESI : 255 (M-H)⁻ |
| 58 | 1-c | 5-Me, 8-Me | -H | -H | FAB : 251 (M-H)⁻ |
| 59 | 1-c | -H | 1-Cl | -H | FAB : 259 (M+H)⁺ |
| 60 | 1-c | -H | 3-Cl | -H | FAB : 259 (M+H)⁺ |
| 61 | 2 | 5-F | -H | -Et | FAB : 271 (M+H)⁺ |
| 62 | 2 | 8-F | -H | -Et | FAB : 271 (M+H)⁺ |
| 63 | 2 | 5-Me | -H | -Me | FAB : 253 (M+H)⁺ |
| 64 | 2 | 6-OMe | -H | -Et | FAB : 283 (Nl+H)⁺ |
| 65 | 2 | 8-OMe | -H | -Et | FAB : 283 (M+H)⁺ |
| 66 | 2 | -H | 1-Cl | -Et | FAB : 287 (M+H)⁺ |
| 67 | 2 | -H | 3-Cl | -Et | FAB : 287 (M+H)⁺ |
| 68 | 4-b | 6-Cl | -H | -CH₂CH=CH₂ | FAB : 299 (M+H)⁺ |
| 69 | 4-b | 8-Cl | -H | -CH₂CH=CH₂ | FAB : 299 (M+H)⁺ |
| 70 | 7-b | 5-CH₂NMe₂ | -H | -Me | FAB : 296 (M+H)⁺ |
| 71 | 8-a | 5-CH₂OAc | -H | -Me | FAB : 311 (M+H)⁺ |
| 72 | 8-c | 5-CH₂OMe | -H | -Me | FAB : 283 (M+H)⁺ |

**Table 3**

| | | | | | | |
|---|---|---|---|---|---|---|
| 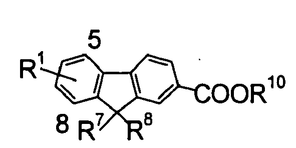 | | | | | | |

| REx | RSyn | R¹ | R⁷ | R⁸ | R¹⁰ | Dat |
|---|---|---|---|---|---|---|
| 73 | 4-a | -H | -Et | -OH | -H | FAB : 253 (M-H)⁻ |
| 74 | 4-a | -H | -Bu | -OH | -H | ESI: 281 (M-H)⁻ |
| 75 | 4-a | 5-F | -Me | -OH | -H | FAB : 257 (M-H)⁻ |
| 76 | 4-a | 5-Me | -Me | -OH | -H | EI : 254 (M)⁺ |
| 77 | 4-a | 5-Et | -Me | -OH | -H | FAB : 267 (M-H)⁻ |
| 78 | 4-a | 5-CF₃ | -Me | -OH | -H | FAB : 307 (M-H)⁻ |
| 79 | 4-a | 8-Me | -Me | -OH | -H | FAB : 253 (M-H)⁻ |
| 80 | 4-a | 5-F, 8-Me | -Me | -OH | -H | FAB : 271 (M-H)⁻ |
| 81 | 4-a | 5-Me, 8-Me | -Me | -OH | -H | FAB : 267 (M-H)⁻ |
| 82 | 4-b | -H | -Et | -OH | -Me | FAB : 269 (M+H)⁺ |
| 83 | 4-b | 5-F | -Me | -OH | -Me | EI : 272 (M)⁺ |
| 84 | 4-b | 8-Me | -Me | -OH | -Me | EI : 268 (M)⁺ |
| 85 | 4-b | 5-F, 8-Me | -Me | -OH | -Me | FAB : 285 (M-H)⁻ |
| 86 | 4-c | -H | -Et | -OMe | -Me | FAB : 283 (M+H)⁺ |
| 87 | 4-c | 5-F | -Me | -OMe | -Me | FAB : 287 (M+H)⁺ |
| 88 | 4-c | 8-Me | -Me | -OMe | -Me | FAB : 283 (M+H)⁺ |
| 89 | 4-c | 5-F, 8-Me | -Me | -OMe | -Me | FAB : 301 (M+H)⁺ |
| 90 | 5 | -H | -Me | -O(CH₂)₂OMe | -Me | EI : 312 (M)⁺ |
| 91 | 7-b | 5-F, 8-CH₂-NMe₂ | -Me | -OMe | -Me | FAB : 344 (M+H)⁺ |
| 92 | 7-b | 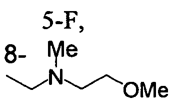 | -Me | -OMe | -Me | FAB : 388 (M+H)⁺ |
| 93 | 14-e | -H | -CH₂OMe | -CH₂OMe | -H | ESI : 297 (M-H)⁻ |
| 94 | 14-e | -H | -(CH₂)₂OMe | -(CH₂)₂OMe | -H | ESI : 325 (M-H)⁻ |
| 95 | 14-e | -H | -(CH₂)₂OBn | -(CH₂)₂OBn | -H | ESI : 477 (M-H)⁻ |
| 96 | 1-b | -H | -F | -H | -H | FAB : 227 (M-H)⁻ |

**Table 4**

| | | | | | | |
|---|---|---|---|---|---|---|
| 97 | 1-b | -H | -NHAc | -H | -H | FAB : 268 (M+H)⁺ |
| 98 | 1-b | -H | -NHBOC | -H | -H | FAB : 338 (M-H)⁻ |
| 99 | 1-b | -H | -(CH₂)₃OH | -OH | -H | EI : 284 (M)⁺ |
| 100 | 1-b | -H | -CH₂OTBS | -CH₂OTBS | -H | FAB : 499 (M+H)⁺ |

**Table 5**

| | | | | | |
|---|---|---|---|---|---|
| 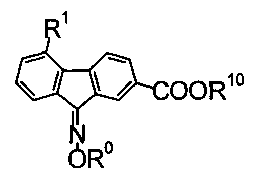 | | | | | |

| REx | RSyn | R⁰ | R¹ | R¹⁰ | Dat |
|---|---|---|---|---|---|
| 101 | 13-a | -H | -F | -H | FAB : 286 (M+H)⁺ |
| 102 | 13-a | -Me | -H | -Pr | FAB : 296 (M+H)⁺ |

**Table 6**

| | | | | | |
|---|---|---|---|---|---|
| 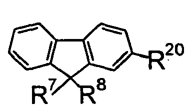 | | | | | |

| REx | RSyn | R⁷ | R⁸ | R²⁰ | Dat |
|---|---|---|---|---|---|
| 103 | 5 | -CH₂OMe | -CH₂OMe | -H | ESI : 255 (M+H)⁺ |
| 104 | 14-b | -(CH₂)₂O (CH₂)₂- | | -Me | FAB : 251 (M+H)⁺ |
| 105 | 14-c | -CH₂OMe | -CH₂OMe | -Br | ESI : 332(M+H)⁺, 334 (M+H+2)⁺ |
| 106 | 14-c | -(CH₂)₂OMe | -(CH₂)₂OMe | -Br | FAB : 361(M+H)⁺, 363 (M+H+2)⁺ |
| 107 | 14-c | -(CH₂)2- | | -Br | EI : 270(M)⁺, 272 (M+2)⁺ |
| 108 | 14-d | -CH₂OMe | -CH₂OMe | -CN | ESI : 280 (M+H)⁺ |
| 109 | 14-d | -(CH₂)₂OMe | -(CH₂)₂OMe | -CN | ESI : 308 (M+H)⁺ |
| 110 | 14-d | -(CH₂)2- | | -CN | FAB : 218 (M+H)⁺ |
| ¹¹¹ | 17-a | -(CH₂)₂OMe | -(CH₂)₂OMe | -H | FAB : 283 (M+H)⁺ |

**Table 7**

| | | | |
|---|---|---|---|
| 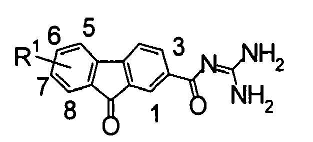 | | | |

| Ex | Syn | R¹ | Dat |
|---|---|---|---|
| 1 | 1 | 5-F | NMR : 7.45-7.55 (3H, m), 7.73 (1H, d, *J=* 7.3 Hz), 8.36 (1H, s). ; FAB : 284 (M+H)⁺ |
| 2 | 2 | -H | NMR : 7.42 (1H, t, *J=* 8.3 Hz), 7.62-7.67 (2H, m), 8 .31 (1H, s). ; FAB : 266 (M+H)⁺ |
| 9 | 9 | 6-Me | NMR : 2.42 (3H, s), 7.22 (1H, d, *J* = 7.3 Hz), 8.29 (1H, s). ; FAB : 280 (M+H)⁺ |
| 10 | 1 | 7-F | NMR : 7.46-7.51 (2H, m), 8.28 (1H, dd, *J* = 7.8, 1.5 Hz), 8.31 (1H, d, *J* = 1.2 Hz). ; FAB : 284 (M+H)⁺ |
| 11 | 1 | 8-F | NMR : 7.21 (1H, ddd, *J=* 9.8, 8.3, 2.5 Hz), 7.70 (1H, dd, *J* = 8.3, 5.3 Hz), 8.31 (1H, s). ; FAB : 284 (M+H)⁺ |
| 12 | 2 | 1-Cl | NMR : 7.43 (1H, t, *J =* 6.8 Hz), 7.62-7.69 (3H, m), 7.75(1H, d, *J=* 7.8 Hz). ; FAB : 300 (M+H)⁺ |
| 13 | 2 | 3-Cl | NMR : 7.44 (1H, t, *J* = 7.3 Hz), 7.79 (1H, s), 7.94(1 H, s). ; FAB : 300 (M+H)⁺ |
| 14 | 2 | 5-Cl | NMR : 7.44 (1H, t, *J=* 7.8 Hz), 8.18 (1H, d, *J=* 7.8 Hz), 8.37 (1H, d, *J*= 1.0 Hz). ; FAB : 300 (M+H)⁺ |
| 15 | 2 | 7-Cl | NMR : 7.64 (1H, d, *J*= 1.9 Hz), 7.84-7.89 (2H, m), 8.32 (1H, s). ; FAB : 300 (M+H)⁺ |
| 16 | 2 | 5-Me | NMR : 2.61 (3H, s), 7.31 (1H, t, *J*= 7.6 Hz), 8.33 (1H, d, *J*= 0.9 Hz). ; FAB : 280 (M+H)⁺ |
| 17 | 2 | 5-Et | NMR: 1.29 (3H, t, J= 7.3 Hz), 7.36 (1H, t, J= 7.3 Hz), 8.33 (1H, d, *J* = 1.4 Hz). ; FAB : 294 (M+H)⁺ |
| 18 | 2 | 7-OMe | NMR: 3.85 (3H, s), 7.69 (1H, d, *J =* 7.8 Hz), 8.26 (1H, s). ; FAB : 296 (M+H)⁺ |
| 19 | 9 | 6-Cl | NMR : 7.64 (1H, d, *J* = 7.3 Hz), 8.02 (1H, d, *J* = 1. 5 Hz), 8.33(1H, s). ; FAB : 300 (M+H)⁺ |
| 20 | 9 | 8-Cl | NMR : 7.40 (1H, d, *J* = 7.8 Hz), 7.63 (1H, t, J= 7.8 Hz), 8.32(1H, s). ; FAB : 300 (M+H)⁺ |
| 21 | 9 | 8-Me | NMR : 2.56 (3H, s), 7.19 (1H, d, J= 7.8 Hz), 8.27 (1H, s). ; FAB : 280 (M+H)⁺ |
| 22 | 9 | 6-OMe | NMR : 3.92 (3H, s), 6.90 (1H, dd, J= 8.3 Hz, 2.0 Hz), 8.26-8.27 (2H, m). ; FAB : 296 (M+H)⁺ |
| 23 | 9 | 8-OMe | NMR : 3.91 (3H, s), 7.61 (1H, t, *J=* 8.1 Hz), 8.26 (1H, s). ; FAB : 296 (M+H)⁺ |
| 24 | 9 | 5-CH₂NMe₂ | NMR : 2.45 (6H, s), 3.66 (2H, s), 8.32 (1H, d, J= 1.0 Hz). ; FAB : 323 (M+H)⁺ |

**Table 8**

| | | | |
|---|---|---|---|
| 25 | 9 | 8-CH₂NMe₂ | NMR : 2.21 (6H, s), 3.85 (2H, s), 8.25 (1H, d, *J=* 1.4 Hz). ; FAB : 323 (M+H)⁺ |
| 26 | 9 | 5-CH₂OH | NMR : 4.84 (2H, d, J= 4.4 Hz), 7.41 (1H, t, J= 7.4 Hz), 8.32 (1H, d, J= 1.4 Hz). ; FAB : 296 (M+H)⁺ |
| 27 | 9 | 8-CH₂OH | NMR : 4.94 (2H, d, *J* = 5.9 Hz), 7.5 (1H, dd, *J* = 7.8, 1.0 Hz), 8.25 (1H, s). ; FAB : 295 (M)⁺ |
| 28 | 9 | 5-CH₂OMe | NMR: 3.42 (3H, s), 4.75 (2H, s), 8.34 (1H, d, *J*= 1.4 Hz). ; FAB : 310 (M+H)⁺ |
| 29 | 9 | 8-CH₂OMe | NMR: 3.40 (3H, s), 4.86 (2H, s), 8.26 (1H, s). ; FAB: 310 (M+H)⁺ |

**Table 9**

| | | | | |
|---|---|---|---|---|
| 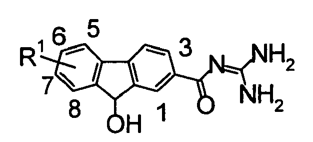 | | | | |

| Ex | Syn | R¹ | Sal | Dat |
|---|---|---|---|---|
| 3 | 3 | -H | HCl | NMR : 5.59 (1H, s), 7.40-7.49 (2H, m), 8.23-8.28 (2H, m). ; FAB : 268 (M+H)⁺ |
| 30 | 3 | 5-F | HCl | NMR : 5.67 (1H, s), 7.30 (1H, t, J= 8.3 Hz), 8.28 (1H, s). ; FAB : 286 (M+H)⁺ |
| 31 | 3 | 7-F | | NMR : 5.49 (1H, d, *J* = 7.3 Hz), 7.18-7.26 (1H, m), 8.32 (1H, s). ; FAB : 286 (M+H)⁺ |
| 32 | 3 | 8-F | | NMR : 5.48 (1H, d, J= 7.8 Hz), 7.14 (1H, ddd, *J* = 9.8, 8.3, 2.4 Hz), 8.33 (1H, s). ; FAB : 286 (M+H)⁺ |
| 33 | 3 | 1-Cl | HCl | NMR : 5.67 (1H, s), 7.43-7.49 (2H, m), 7.65 (1H, dd, J= 6.3, 2.0 Hz). ; FAB : 302 (M+H)⁺ |
| 34 | 3 | 3-Cl | HCl | NMR : 5.56 (1H, s), 7.42-7.48 (2H, m), 8.13 (1H, s). ; FAB : 302 (M+H)⁺ |
| 35 | 3 | 5-Cl | HCl | NMR : 5.63 (1H, s), 7.65 (1H,d, *J*= 6.8 Hz), 8.29-8.31 (2H, m). ; FAB : 302 (M+H)⁺ |
| 36 | 3 | 6-Cl | HCl | NMR : 5.59 (1H, s), 7.47 (1H, dd, *J*= 8.1, 1.7 Hz), 8.08-8.10 (2H, m),. ; FAB : 302 (M+H)⁺ |
| 37 | 3 | 7-Cl | HCl | NMR: 5.59 (1H, d, *J=* 7.3 Hz), 7.65 (1H,s), 8.27 (1H, s). ; FAB : 302 (M+H)⁺ |
| 38 | 3 | 8-Cl | HCl | NMR : 5.70 (1H, s), 7.49 (1H, t, J= 7.8 Hz), 8.25 (1H, s). ; FAB : 302 (M+H)⁺ |
| 39 | 3 | 5-Me | HCl | NMR : 2.67 (3H, s), 5.54 (1H, s), 8.27 (1H, s). ; FAB : 282 (M+H) |

**Table 10**

| | | | | |
|---|---|---|---|---|
| 40 | 3 | 6-Me | HCl | NMR: 2.41 (3H, s), 5.54 (1H, s), 8.22 (1H, s). ; FAB : 282 (M+H) |
| 41 | 3 | 8-Me | HCl | NMR: 2.50 (3H, s), 5.64 (1H, s), 8.20 (1H, s). ; FAB : 282 (M+H) |
| 42 | 3 | 5-Et | | NMR: 1.28 (3H, t, *J=* 7.3 Hz), 5.42 (1H, d, *J=* 7.3 Hz), 8.34 (1H, s). ; FAB : 296 (M+H)⁺ |
| 43 | 3 | 6-OMe | HCl | NMR: 3.85 (3H, s), 5.52 (1H, s), 8.21 (1H, s). ; FAB : 298 (M+H) |
| 44 | 3 | 7-OMe | HCl | NMR: 3.85 (3H, s), 5.53 (1H, s), 8.20 (1H, s).; FAB : 298 (M+H) |
| 45 | 3 | 8-OMe | HCl | NMR :3.89 (3H, s), 5.66 (2H, brs), 8.20 (1H, s).; FAB : 298 (M+H)⁺ |
| 46 | 3 | 5-CH₂NMe₂ | 2HCl | NMR: 2.85 (6H, s), 5.58 (1H, s), 8.32 (1H, s).; FAB : 325 (M+H) |
| 47 | 3 | 8-CH₂NMe₂ | 2HCl | NMR: 2.79 and 2.85 (6H, s and s), 5.99 (1H, s), 8.33 (1H, s). ; FAB : 325 (M+H)⁺ |
| 48 | 3 | 5-CH₂OH | HCl | NMR: 4.89 (2H, s), 5.56 (1H, s), 8.24 (1H, s). ; FAB : 298 (M+H) |
| 49 | 3 | 8-CH₂OH | HCl | NMR: 4.84 (2H, s), 5.69 (1H, s), 7.44-7.50 (2H, m).; FAB : 298 (M+H)⁺ |
| 50 | 3 | 5-CH₂OMe | HCl | NMR : 3.39 (3H_{,} s), 5.57 (1H, s), 8.23 (1H, s). ; FAB : 312 (M+H)⁺ |
| 51 | 3 | 8-CH₂OMe | HCl | NMR: 3.39 (3H, s), 5.69 (1H, s), 8.24 (1H, s). ; FAB : 312 (M+H)⁺ |

**Table 11**

| | | | | | |
|---|---|---|---|---|---|
| 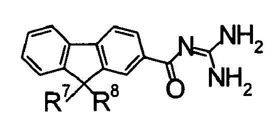 | | | | | |

| Ex | Syn | R⁷ | R⁸ | Sal | Dat |
|---|---|---|---|---|---|
| 4 | 4 | -H | -Cl | | NMR : 6.26 (1H, s), 7.40-7.45 (1H, m), 8.36 (1H, s). ; FAB : 286 (M+H)⁺ |
| 5 | 5 | -H | -NH₂ | 2HCl | NMR : 5.54 (1H, brs), 7.54 (1H, dt, *J* = 7.3, 1.0 Hz), 8.61 (1H, s). ; FAB : 267 (M+H⁺ |
| 6 | 6 | -CH₂OH | -CH₂OH | HCl | NMR : .3.78 (4H, s), 7.38-7.46 (2H, m), 8.34 (1H, d, *J*= 0.9 Hz). ; FAB : 312 (M+H)⁺ |

**Table 12**

| | | | | | |
|---|---|---|---|---|---|
| 52 | 2 | -H | -H | | NMR: 3.96 (2H, s), 7.31-7.42 (2H, m), 8.31 (1H, s). ; FAB : 252 (M+H)⁺ |
| 53 | 2 | -H | -Me | HCl | NMR : 1.54 (3H, d, *J*= 7.3 Hz), 4.07 (1H, q, *J*= 7.3 Hz), 8.49 (1H, s). ; FAB : 266 (M+H)⁺ |
| 54 | 2 | -H | -F | HCl | NMR: 6.60 (1H, d, *J=* 52.7 Hz), 7.49 (1H, t, *J*= 7.8 Hz), 8.36 (1H, s). ; FAB : 270 (M+H)⁺ |
| 55 | 2 | -H | -NHCOCH₃ | HCl | NMR : 1.98 (3H, s), 6.11 (1H, d, *J*= 7.3 Hz), 8.14 (1H, s). ; FAB : 309 (M+H)⁺ |
| 56 | 2 | -OH | -Me | HCl | NMR : 1.64 (3H, s), 7.39-7.43 (2H, m), 8.26 (1H, d, *J* = 1.4 Hz). ; FAB : 282 (M+H)⁺ |
| 56a | | -OH* | -Me* | HCl | RT : 7.39, Proc.A; FAB : 282 (M+H)⁺ |
| 56b | | -OH* | -Me* | HCl | RT : 11.98, Proc.A ; FAB : 282 (M+H)⁺ |
| 57 | 1 | -OH | -Et | HCl | NMR : 0.42 (3H, t, J= 7.3 Hz), 2.04-2.18 (2H, m), 8.19 (1H, d, *J* = 1.0 Hz).; FAB : 296 (M+H)⁺ |
| 58 | 2 | -OH | -Bu | HCl | NMR : 0.65-0.75 (5H, m), 7.39-7.44 (2H, m), 8.18 (1H, brs) ; FAB : 324 (M+H)⁺ |
| 59 | 2 | -S(CH₂)₂S- | | HCl | NMR : 3.83-3.90 (4H, m), 7.46-7.50 (2H, m), 8.57 (3H, m) ; FAB : 342 (M+H)⁺ |
| 60 | 9 | -O(CH)₃- | | HCl | NMR : 2.25-2.44 (4H, m), 7.39-7.46 (2H, m), 8.37 (1H, d, J= 1.5 Hz). ; FAB : 308 (M+H)⁺ |
| 60a | | -O(CH₂)₃-* -O(CH₂)₃-* | | HCl | RT : 14.34, Proc.B ; FAB : 308 (M+H)⁺ |
| 60b | | -O(CH₂)₃-* -O(CH₂)₃-* | | HCl | RT : 18.66, Proc.B ; FAB : 308 (M+H)⁺ |
| 61 | 1 | -CH₂OMe | -CH₂OMe | HCl | NMR : 3.17 (6H, s), 3.45 (4H, s), 8.39 (1H, d, *J*= 1.0 Hz). ; FAB : 340 (M+H)⁺ |
| 62 | 1 | -(CH₂)₂OMe | -(CH₂)₂OMe | HCl | NMR: 2.33-2.48 (4H, m), 2.87 (6H, s), 8.56 (1H, s).; FAB:368 (M+H)⁺ |
| 63 | 1 | -(CH₂)₂- | | HCl | NMR : 1.81-1.86 (2H, m), 7.27-7.31 (1H, m), 8.20 (1H, d, *J*= 1.4 Hz).; FAB : 278 (M+H)⁺ |

**Table 13**

| | | | | | |
|---|---|---|---|---|---|
| 64 | 1 | -(CH₂)₄- | | HCl | NMR: 1.94-2.02 (2H, m), 7.3 8-7.45 (2H, m), 8.40 (1H, s),. ; FAB : 306 (M+H)⁺ |
| 65 | 1 | -OH | 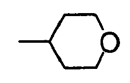 | HCl | NMR : 1.37 (2H, d, *J=* 1.7 Hz), 7.40-7.46 (2H, m), 8.12 (1H, s).; FAB : 352 (M+H)⁺ |
| 66 | 1 | -H | 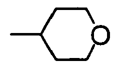 | HCl | NMR : 4.12 (1H, d, *J* =3.0 Hz), 7.43-7.48 (2H, m), 8.30 (1H, s). ; FAB : 335 (M+H)⁺ |
| 67 | 1 | 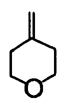 | | HCl | NMR : 3.21-3.24 (2H, m), 7.43-7.45 (2H, m), 8.57 (1H, s). ; FAB : 334 (M+H)⁺ |
| 68 | 9 | -OH | -OCH₂OMe | | NMR : 1.64 (3H, s), 3.04 (3H, s), 8.24 (1H, d, J= 1.5 Hz). ; FAB : 326 (M+H)⁺ |
| 69 | 9 | -OH | -O(CH₂)₂OMe | HCl | NMR : 1.67 (3H, s), 3.13 (3H, s), 8.22-8.24 (3H, m). ; FAB : 340 (M+H)⁺ |
| 70 | 9 | -OH | -O(CH₂)₃OH | HCl | NMR : 0.87-0.94 (2H, m), 3.19 (2H, t, J= 6.6 Hz), 8.17 (1H, s). ; FAB : 326(M+H)⁺ |
| 71 | 9 | -OMe | -Me | HCl | NMR : 1.67 (3H, s), 2.65 (3H, s), 8.30 (1H, d, J= 1.5 Hz). ; FAB : 296 (M+H)⁺ |
| 72 | 9 | -OMe | -Et | HCl | NMR : 0.43 (3H, t, J= 7.6 Hz), 2.68 (3H, s), 8.17 (1H, d, *J* = 1.5 Hz). ; FAB : 310(M+H)⁺ |
| 73 | 9 | -OMe | -OMe | | NMR : 3.29 (6H, s), 7.36 (1H, dt, *J=* 0.9, 7.6 Hz), 8.24 (1H, s). ; FAB : 312 (M+H)⁺ |
| 74 | 9 | -O(CH₂)₂O- | | | NMR : 4.33-4.42 (4H, m), 7.32 (1H, dt, *J=* 1.0, 7.3 Hz), 8.21 (1H, s). ; FAB : 310 (M+H)⁺ |
| 75 | 9 | -(CH₂)₂O(CH₂)₂- | | HCl | NMR : 1.76 (2H, dt, J= 13.7 Hz, 4.9 Hz), 4.06-4.11 (4H, m), 8.52 (1H, s). ; FAB : 322 (M+H)⁺ |
| 76 | 9 | -(CH₂)₂NMe(CH₂)₂- | | 2HCl | NMR : 1.63-1.71 (2H, m), 2.54 (3H, s), 8.92 (1H, s). ; ESI : 335 (M+H)⁺ |

**Table 14**

| | | | | | | |
|---|---|---|---|---|---|---|
| 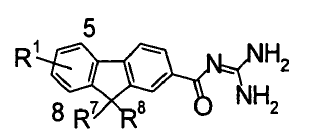 | | | | | | |

| Ex | Syn | R¹ | R⁷ | R⁸ | Sal | Dat |
|---|---|---|---|---|---|---|
| 77 | 1 | 5-F | -OH | -Me | HCl | NMR : 1.69 (3H, s), 7.25-7.30 (1H, m), 8.29 (1H, d, *J* = 1.5 Hz).; FAB : 300 (M+H)⁺ |
| 78a | | 5-F | -OH* | -Me* | HCl | RT : 3.97, Proc.C ; FAB **:** 300 (M+H)⁺ |
| 78b | | 5-F | -OH* | -Me* | HCl | RT : 5.78, Proc.C ; FAB **:** 300 (M+H)⁺ |
| 79 | 1 | 5-Me | -OH | -Me | HCl | NMR: 1.62 (3H, s), 2.66 (3H, s), 8.28 (1H, s). ; FAB : 296 (M+H)⁺ |
| 80 | 1 | 5-Et | -OH | -Me | HCl . | NMR : 1.29 (3H, t, J= 7.3 Hz), 1.61 (3H,s), 8.26 (1H, d, *J =* 1.9 Hz). ; FAB : 310 (M+H)⁺ |
| 81 | 1 | 5-CF₃ | -OH | -Me | HCl | NMR: 1.67 (3H, s), 7.65 (1H, t, *J* =7.8 Hz), 8.37 (1H, d, *J* = 1.4 Hz). ; FAB : 350 (M+H)⁺ |
| 82 | 1 | 8-Me | -OH | -Me | HCl | NMR : 1.69 (3H, s), 2.55 (3H, s), 8.23 (1H, d, J= 1.5 Hz). ; FAB : 296 (M+H)⁺ |
| 83 | 1 | 5-Me, 8-Me | -OH | -Me | HCl | NMR : 1.67 (3H, s), 2.53 (3H, s), 2.67 (3H, s) ; FAB : 310 (M+H)⁺ |
| 84 | 2 | 5-F, 8-Me | -OH | -Me | HCl | NMR : 1.70 (3H, s), 2.53 (3H, s), 8.29 (1H, d, *J* = 1.5 Hz). ; FAB : 314 (M+H)⁺ |
| 85 | 2 | 5-F | -S(CH₂)₂S- | | HCl | NMR: 3.87 (1H, dt, J= 11.0, 3.9 Hz), 3.89 (1H, dt, *J =* 11.0, 5.8 Hz), 8.64 (1H, d, *J* = 1.5 Hz). ; FAB : 360 (M+H)⁺ |
| 86 | 1 | 5-F | -(CH₂)₂O (CH₂)₂- | | HCl | NMR : 1.74-1.80 (2H, m), 7.32 (1H, t, *J* = 8.3 Hz), 8.54 (1H, s). ; FAB : 340(M+H)⁺ |
| 87 | 9 | 5-F | -OMe | -Me | HCl | NMR: 1.69 (3H, s), 2.67 (3H, s), 8.32 (1H,d,*J* = 1.5 Hz).;FAB: 314(M+H)⁺ |
| 88 | 9 | 8-Me | -OMe | -Me | HCl | NMR : 1.72 (3H, s), 2.68 (3H, s), 8.22 (1H, d, *J =* 1.5 Hz). ; FAB 310 (M+H)⁺ |

**Table 15**

| | | | | | | |
|---|---|---|---|---|---|---|
| 89 | 9 | 5-F, 8-CH₂NMe₂ | -OMe | -Me | 2HCl | NMR : 1.81 (3H, s), 2.76 (3H, s), 8.42 (1H, s). ; FAB : 371 (M+H)⁺ |
| 90 | 9 | 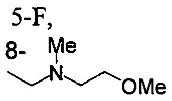 | -OMe | -Me | 2HCl | NMR : 1.81 and 1.82 (3H, s and s), 2.77 and 2.78 (3H, s and s), 2.84 and 2.85 (3H, s and s). ; FAB : 415 (M+H)⁺ |

**Table 16**

| | | | | | |
|---|---|---|---|---|---|
| 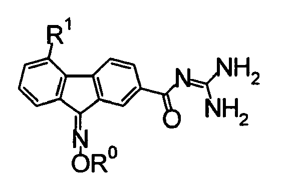 | | | | | |

| Ex | Syn | R⁰ | R¹ | Sal | Dat |
|---|---|---|---|---|---|
| 7 | 7 | -H | -CH₂OH | HCl | NMR : 4.91 and 4.93 (2H, s and s), 7.45-7.56 (2H, m), 7.63-7.74 (2H, m). ; FAB : 311 (M+H)⁺ |
| 91 | 9 | -H | -H | HCl | NMR : 7.51 (1H, dt, J= 7.3, 1.0 Hz), 7.59 (1H, dt, *J*= 7.3, 1.0 Hz), 8.36 (1H, s). ; FAB : 281 (M+H)⁺ |
| 92 | 9 | -Me | -H | HCl | NMR: 4.24 and 4.25 (3H, s and s), 7.44-7.65 (2H, m), 8.22-8.36 (2H, m Hz). ; FAB : 295 (M+H)⁺ |
| 93 | 9 | -H | -F | | NMR : 7.29-7.61 (2H, m), 7.78-7.84 (1H, m), 8.18-8.29 (2H, m). ; FAB : 299 (M+H)⁺ |
| 94 | 7 | -H | -CH₂NMe₂ | 2HCl | NMR : 2.87 (6H, s), 4.83 (2H, s), 7.44-7.65 (1H, m). ; FAB : 338 (M+H)⁺ |
| 95 | 7. | -H | -CH₂OMe | HCl | NMR : 3.41 and 3.42 (3H, s and s), 4.82 and 4.84 (2H, s and s), 7.43-7.60 (2H, m). ; FAB : 325 (M+H)⁺ |

**Table 17**

| | | | | | |
|---|---|---|---|---|---|
| 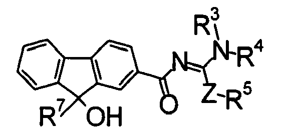 | | | | | |

| Ex | Syn | R⁷ | 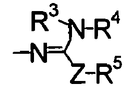 | Sal | Dat |
|---|---|---|---|---|---|
| 8 | 8 | H | 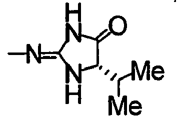 | | NMR: 0.94-0.99 (6H, m), 4.20 (1H, d, *J=* 4.0 Hz), 5.54 (1H, s) ; FAB : 350 (M+H)⁺ |
| 96 | 2 | Me | 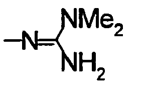 | HCl | NMR: 1.63 (3H, s), 3.18 (6H,s), 8.16 (1H, s) ; FAB : 310 (M+H)⁺ |
| 97 | 2 | Me | 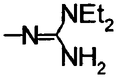 | HCl | NMR : 1.21 (6H, t, J= 6.8 Hz), 1.63 (3H, s), 8.16 (1H, s) ; FAB : 338 (M+H)⁺ |
| 98 | 2 | Me | 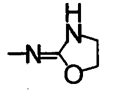 | | NMR : 1.59 (3H, s), 3.80 (2H, t, *J*= 8.8 Hz), 8.24 (1H, brs); FAB : 309 (M+H)⁺ |
| 99 | 2 | Me | 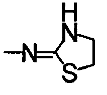 | HCl | NMR: 1.61 (3H, s), 3.42 (2H, t, *J=* 8.8 Hz), 8.27 (1H, brs). ; FAB : 325 (M+H)⁺ |
| 100 | 2 | Me | 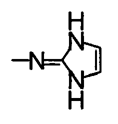 | | NMR : 1.62 (3H, s), 6.83 (2H, s), 8.26 (1H, d, *J* = 0.8 Hz). ; FAB : 306 (M+H)⁺ |
| 101 | 2 | Me | 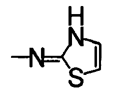 | | NMR: 1.64 (3H, s), 7.29 (1H, d, *J*= 3.6 Hz), 8.31 (1H, d, *J =* 1.6 Hz). ; FAB : 323 (M+H)⁺ |
| 102 | 2 | Me | 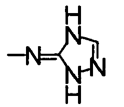 | | NMR : 1.61 (3H, s), 7.91 (1H, d, *J=* 7.6 Hz), 8.23 (1H, d, *J* = 1.6 Hz).; FAB : 307 (M+H)⁺ |
| 103 | 2 | Me | 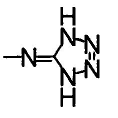 | | NMR : 1.64 (3H, s), 7.94 (1H, d, *J=* 7.8 Hz), 8.23 (1H, d, *J =* 1.6 Hz). ; FAB : 308 (M+H)⁺ |
| 104 | 2 | Me | -NH₂ | | NMR : 1.60 (3H, s), 7.90 (1H, dd, J= 8.0, 1.6 Hz), 8.07 (1H, d, *J* = 1.2 Hz). ; FAB: 240 (M+H) |

**Table 18**

| Ex | Syn | Str | Sal | Dat |
|---|---|---|---|---|
| 105 | 2 | 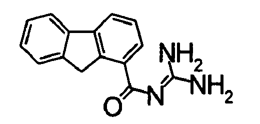 | HCl | JMR : 4.26 (2H, s), 7.36-7.46 2H, m), 8.25 (1H, d, J= 7.8 Hz). ; FAB : 252 (M+H)⁺ |
| 106 | 2 | 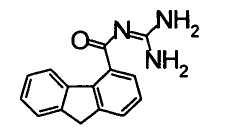 | HCl | NMR:4.01 (2H, s), 7.36-7.44 2H, m), 7.83-7.91 (1H, m). ; FAB : 252 (M+H)⁺ |
| 107 | 2 | 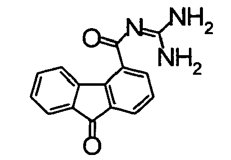 | | NMR : 7.33-7.39 (2H, m), 7.75 1H, dd, J = 8.0, 1.2 Hz), 8.04 1H, d, *J* = 7.6 Hz). ; FAB : 266 M+H)⁺ |
| 108 | 2 | 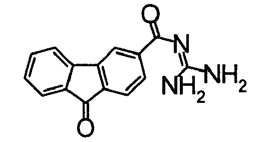 | HCl | NMR : 7.47 (1H, t, *J* = 7.4 Hz), 7.68-7.73 (2H, m), 8.67 (1H, s). ; FAB : 266 (M+H)⁺ |
| 109 | 3 | 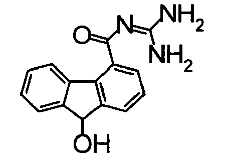 | HCl | NMR : 5.51 (1H, s), 7.35-7.44 2H, m), 7.83 (1H, d, *J* = 7.1 Hz). ; FAB : 268 (M+H)⁺ |
| 110 | 3 | 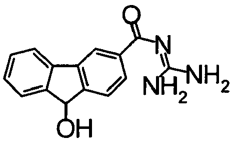 | HCl | NMR : 5.59 (1H, s), 7.39 (1H, t, *J*= 7.4 Hz), 8.74 (1H, s). ; FAB : 268 (M+H)⁺ |

**Table 19**

| | | | | | |
|---|---|---|---|---|---|
| 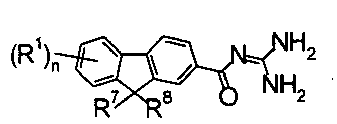 | | | | | |

| Cmp | 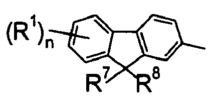 | Cmp | 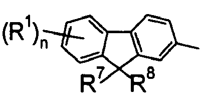 | Cmp | 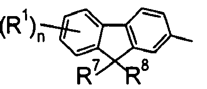 |
|---|---|---|---|---|---|
| 1 | 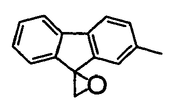 | 8 | 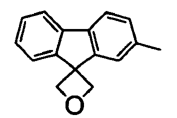 | 15 | 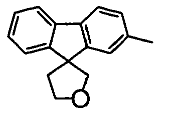 |
| 2 | 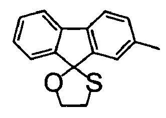 | 9 | 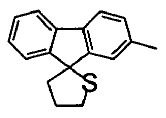 | 16 | 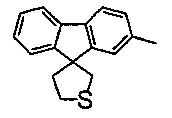 |
| 3 | 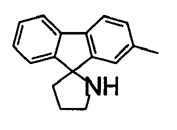 | 10 | 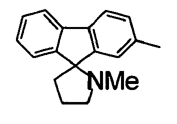 | 17 | 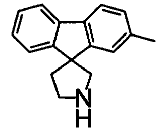 |
| 4 | 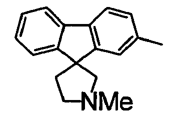 | 11 | 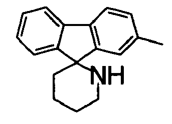 | 18 | 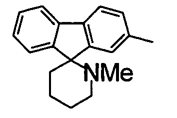 |
| 5 | 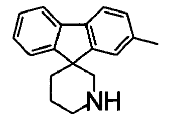 | 12 | 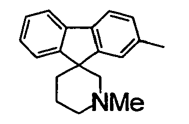 | 19 | 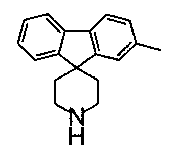 |
| 6 | 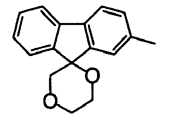 | 13 | 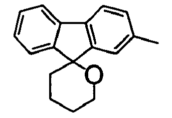 | 20 | 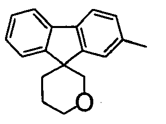 |
| 7 | 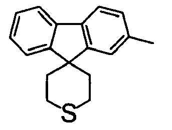 | 14 | 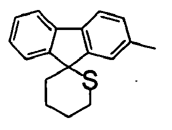 | 21 | 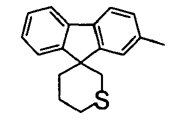 |

**Table 20**

| | | | | | |
|---|---|---|---|---|---|
| 22 | 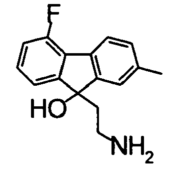 | 29 | 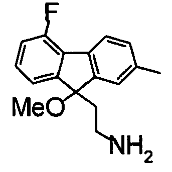 | 36 | 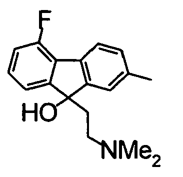 |
| 23 | 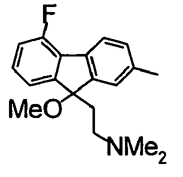 | 30 | 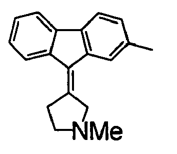 | 37 | 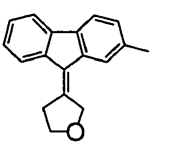 |
| 24 | 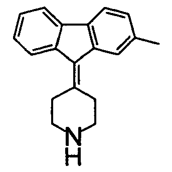 | 31 | 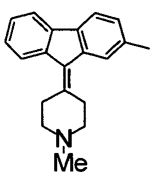 | 38 | 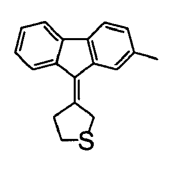 |
| 25 | 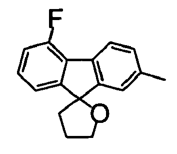 | 32 | 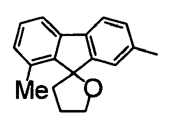 | 39 | 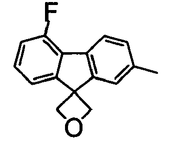 |
| 26 | 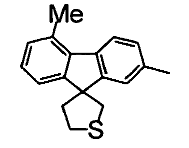 | 33 | 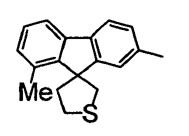 | 40 | 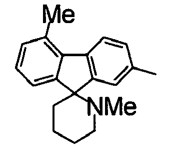 |
| 27 | 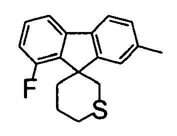 | 34 | 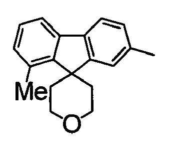 | 41 | 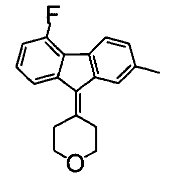 |
| 28 | 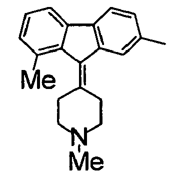 | 35 | 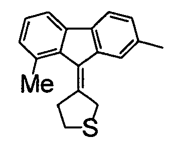 | 42 | 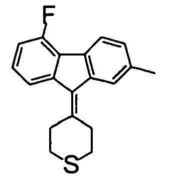 |

### EXAMPLES

The following describes pharmacological actions of the medicament for IBS of the invention based on examples. In addition, results of the test on receptor affinity are shown in reference examples.

### Reference Example 1 Binding test of 5-HT_{2B} receptor

### (i) Preparation of membrane preparation

A human 5-HT_{2B} receptor expressing cell was prepared in accordance with a reference (FEBS Letters (1994) 342, 85 - 90). HEK293-ENBA cell was used as the gene transferring cell.
Cultured HEK293-EBNA cells expressing human 5-HT_{2B} receptor were washed with PBS(-). The cells were scraped in the presence of PBS(-), and the cells were recovered by centrifugation (1,000 rpm, 10 min, 4°C). They were homogenized using Polytron (PTA 10-TS) in the presence of 5 mM Tris-HCl (pH 7.4) buffer and centrifuged (40,000 x g, 10 min, 4°C). They were suspended using a homogenizer in the presence of 50 mM Tris-HCl (pH 7.4) buffer. They were subjected to centrifugation (40,000 x g, 10 min, 4°C), suspended in 50 mM Tris-HCl (pH 7.4) and stored at -80°C.

### (ii) Receptor binding test

A total volume of 500 µl containing 50 mM Tris-HCl-4 mM CaCl₂ (pH 7.4) buffer, the human 5-HT_{2B} receptor expressing HEK293-EBNA cell membrane preparation and a radio ligand [³H]Mesulergine (3.1 TBq/mmol) was incubated at 25°C for 1 hour. The compound was dissolved in 100% DMSO and diluted to respective concentrations. Nonspecific binding was defined as the binding quantity in the presence of 1 µM ritanserin, and the result of subtracting the nonspecific binding quantity from the total binding quantity was defined as the specific binding quantity. This was mixed with 4 ml of 50 mM Tris-HCl buffer (pH 7.4) and filtered under a reduced pressure using a GF/B glass filter, and the filter was washed (4 ml x 3) with the same buffer. The glass filter was soaked in 5 ml of a liquid scintillator (Aquasol-2) and the radioactivity was measured using a liquid scintillation counter. Concentration of the compound which inhibits 50% of the receptor binding, IC₅₀ value, was obtained by nonlinear regression analysis using SAS (ver. 6.11), and the Ki value which represents its affinity for the receptor was calculated using the formula of Cheng & Prussoff; Ki = IC₅₀/(1 + [L]/[Kd]) ([L]: ligand concentration, [Kd]: dissociation constant).
The compound of Preparation 3 which is described above showed a Ki value of 1.8 nM. Also, the compounds of Preparation 4, 7, 8, 34, 38, 56, 56a, 56b, 59, 60, 60a, 60b, 63, 71, 72, 77, 78a, 78b, 85 and 87 showed Ki values of from 0.1 to 350 nM.

### Reference Example 2 (2) Binding test of 5-HT₇ receptor

### (i) Preparation of membrane preparation

A human 5-HT₇ receptor expressing cell was prepared in accordance with references (J Biol. Chem. (1993) 268, 31, 23422 - 23426, Br. J. Phaemacol. (1997) 122, 126 - 132). CHO cell was used as the gene transferring cell.
Cultured CHO cells expressing human 5-HT₇ receptor were washed with PBS(-). The cells were scraped in the presence of PBS(-), and the cells were recovered by centrifugation (1,000 rpm, 10 min, 4°C). They were homogenized using Polytron (PTA 10-TS) in the presence of 5 mM Tris-HCl (pH 7.4) buffer and centrifuged (40,000 x g, 10 min, 4°C). They were suspended using a homogenizer in the presence of 50 mM Tris-HCl (pH 7.4) buffer. They were subjected to centrifugation (40,000 x g, 10 min, 4°C), suspended in 50 mM Tris-HCl (pH 7.4) and stored at -80°C.

### (ii) Receptor binding test

A total volume of 500 µl containing 50 mM Tris-HCl-4 mM CaCl₂ (pH 7.4) buffer, the human 5-HT₇ receptor expressing CHO cell membrane preparation and a radio ligand [³H]5-HT (3.40 TBq/mmol) was incubated at 25°C for 1 hour. The compound was dissolved in 100% DMSO and diluted to respective concentrations. Nonspecific binding was defined as the binding quantity in the presence of 10 µM metergoline, and the result of subtracting the nonspecific binding quantity from the total binding quantity was defined as the specific binding quantity. This was mixed with 4 ml of 50 mM Tris-HCl buffer (pH 7.4) and filtered under a reduced pressure using a GF/B glass filter, and the filter was washed (4 ml x 3) with the same buffer. The glass filter was soaked in 5 ml of a liquid scintillator (Aquasol-2) and the radioactivity was measured using a liquid scintillation counter. Concentration of the compound which inhibits 50% of the receptor binding, IC₅₀ value, was obtained by nonlinear regression analysis using SAS (ver. 6.11), and the Ki value which represents its affinity for the receptor was calculated using the formula of Cheng & Prussoff; Ki = IC₅₀/(1 + [L]/[Kd]) ([L]: ligand concentration, [Kd]: dissociation constant).
The compound of Preparation 3 which is described above showed a Ki value of 17.6 nM. Also, the compounds of Preparation 4, 7, 8, 34, 38, 56, 56a, 56b, 59, 60, 60a, 60b, 63, 71, 72, 77, 78a, 78b, 85 and 87 showed Ki values of from 0.4 to 310 nM.

### Reference Example 3 Affinity for other receptors

Affinity of the compound of Preparation 3 for 5-HT_{1A}, 5-HT_{1B}, 5-HT_{2A}, 5-HT_{2C}, 5-HT₃, 5-HT₄, 5-HT₆, α₁, M₁ and D₂ receptors was verified using conventionally known techniques (Journal of Neurochemistry (1986) 47, 529 - 540; Molecular Pharmacology (1982) 21, 301 - 314; European Journal of Pharmacology (1985) 106, 539 - 546; Journal of Pharmacology Experimental Therapy (1992) 263, 1127 - 1132; British Journal of Pharmacology (1993) 109, 618 - 624; Molecular Pharmacology (1993) 43, 320 - 327; Molecular Pharmacology (1989) 35, 324 - 330; Cellular Molecular Neurobiology (1988) 8, 181 - 191; European Journal of Pharmacology (1988) 173, 177 - 182). As a result, IC₅₀ value of this compound was 1 µM or more on all of the 5-HT_{1A}, 5-HT_{1B}, 5-HT_{2A}, 5-HT_{2C}, 5-HT₃, 5-HT₄, 5-HT₆, α₁, M₁ and D₂ receptors. In addition, when the affinity for each of α₁, M₁ and D₂ receptors was verified using the aforementioned technique on the compounds of Preparation 56, 59, 60, 71, 72, 77 and 85 which are described above, 5-HT_{2B} and 5-HT₇ receptor selectivity of these compounds against α₁, M₁ and D₂ receptors was 100 times or more.
Based on the above results, the compound of this production example was a dual antagonistic compound for 5-HT_{2B} and 5-HT₇ receptors having selective binding affinities for both of the 5-HT_{2B} and 5-HT₇ receptors.
In this connection, affinities of the RS-127445 and SB-269970 described in the following Inventive Example 1 for the respective receptors are conventionally known, and regarding RS-127445, it has been reported for example in British Journal of Pharmacology (1999) 127, 1075 - 1082 that said compound has a pKi value of 9.5 for 5-HT_{2B} receptor and is 1,000 times or more selective for 5-HT_{2B} receptor in comparison with 5-HT_{1A}, 5-HT_{1B}, 5-HT_{2A}, 5-HT_{2C}, 5-HT₃, 5-HT₆, 5-HT₇, α₁, M₁ and D₂ receptors. In addition, regarding SB-269970, it has been reported for example in J. Med. Chem. (2000) 43, 342 - 345 that said compound has a pKi value of 8.9 for 5-HT₇ receptor and is 250 times or more selective for 5-HT₇ receptor in comparison with 5-HT_{1A}, 5-HT_{1B}, 5-HT_{2A}, 5-HT_{2B}, 5-HT_{2C}, 5-HT₄, 5-HT₆, α₁ and D₂ receptors.

### Example 1 Effect of compounds to be tested on the suppression of evacuation at the time of restriction stress loading

IBS-treating effect of the medicine of the invention was evaluated using a test method in which the amount of evacuations is measured by applying a restriction stress to rats (cf. J. Pharmacol. Exp. Ther. (1992) 261, 297 - 303). This test is an animal model by which it is known that a diarrhea type IBS remedy, 5-HT₃ receptor antagonist, shows its efficacy.
Test method
Each drug to be tested was administered to male Wister rats (body weight 250 to 320 g, 10 animals for each group), and a restraint stress was loaded 30 minutes thereafter. A restraint stress cage (KN-468, 265 mm in width x 95 mm in length x 200 mm in height, mfd. by Natsume Seisakusho, Tokyo) was used for the restraint stress loading, and the number of excreted stool was counted 1 hour after the stress loading.
Values of the number of stools measured at the time of the addition and no addition of each compound are shown in Fig. 1 to Fig. 4. In these drawings, the axis of abscissa shows dose of the compound, and the axis of ordinate the number of evacuations per hour. The control means the number of stools at the time of no stress loading, namely the reference value.
As shown in Fig. 1, a 5-HT_{2B} selective antagonistic compound, RS-127445, did not show the evacuation suppressing action even when a dose of 10 mg/kg was orally administered (to be referred to as p.o. hereinafter).
In addition, as shown in Fig. 2, a 5-HT₇ selective antagonistic compound, SB-269970, also did not show the evacuation suppressing action even when a dose of 10 mg/kg (p.o.).
In spite of the possession of strong affinities for receptors as described in Reference Example 3, each of the compounds does not show the suppressing action even by the 10 mg/kg administration.
On the other hand, as shown in Fig. 3, it was found that a synergistic effect can be obtained when both compounds RS-127445 and SB-269970 are simultaneously administered. That is, as shown in Fig. 1 and Fig. 2, it was revealed that RS-127445 and SB-269970 do not show the reaction even at a dose of 10 mg/kg (p.o.) when used each independently, but in the case of the simultaneous administration of both compounds, they show significant suppression action starting at a dose of 1 mg/kg (p.o.).
This effect was the same when the compound of Production Example 3 having both of the selective 5-HT_{2B} receptor antagonism and 5-HT₇ receptor antagonism was used, and as shown in Fig. 4, this compound showed significant suppression action when a dose of 3 mg/kg was intraperitoneally administered (to be referred to as i.p. hereinafter).
In addition, the compound of Production Example 60b showed the suppression action similar to the level of Production Example 3 at a dose of 3 mg/kg (p.o.).
Based on the above results, it was confirmed that the 5-HT_{2B} receptor selective antagonistic compound and 5-HT₇ receptor selective antagonistic compound do not express sufficient pharmacological action when used independently. In addition, it was shown that concomitant use of a medicine having 5-HT_{2B} receptor antagonism and a medicine having 5-HT₇ receptor antagonism, and a medicine having both of the actions, can exert superior IBS treating effect which cannot be achieved by one of the selective receptor antagonists.

### INDUSTRIAL APPLICABILITY

The IBS remedy of the invention exerts superior IBS-treating effect by simultaneously inhibiting the functions of 5-HT_{2B} and 5-HT₇ receptors, and the side effects reported on the conventional drugs, caused by the receptor antagonism of other than the 5-HT_{2B} and 5-HT₇ receptors, are reduced, so that this is useful as an IBS remedy which is excellent in the effect and has high safety.

## Claims

1. A medicament for irritable bowel syndrome, which comprises a selective dual antagonist for 5-HT_{2B} and 5-HT₇ receptors as an active ingredient.

2. The medicament for irritable bowel syndrome described in claim 1, wherein its binding affinities for 5-HT_{2B} and 5-HT₇ receptors are 100 times or more of those of each of α₁, M₁, D₂, 5-HT_{1A}, 5-HT_{1B}, 5-HT_{2A}, 5-HT_{2C}, 5-HT₃, 5-HT₄ and 5-HT₆.

3. The medicament for irritable bowel syndrome described in claim 1, wherein the selective dual antagonist for 5-HT_{2B} and 5-HT₇ receptors comprises a dual antagonistic compound for 5-HT_{2B} and 5-HT₇ receptors having selective binding affinity for both of the 5-HT_{2B} and 5-HT₇ receptors.

4. The medicament for irritable bowel syndrome described in claim 3, wherein the dual antagonistic compound for 5-HT_{2B} and 5-HT₇ receptors having selective binding affinity for both of the 5-HT_{2B} and 5-HT₇ receptors is a fluorene derivative represented by the following general formula (I) or a salt thereof; 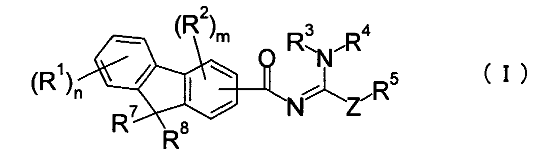 wherein the symbols have the following significances.
R¹ and R² are the same or different from each other and each represents -R⁰, lower alkenyl, lower alkynyl, halogen, -OH, -O-R⁰, -O-CO-R⁰, -NH₂, -NR⁶-R⁰, -CN, -NO₂, -CHO, -CONH₂, -CO-NR⁶-R⁰, -CO₂H, -CO₂-R⁰, -CO-R⁰, -NR⁶-CO-R⁰, -NR⁶-CO₂-R⁰, -O-CO-NR⁶-R⁰, -SH, -S(O)ₚ-R⁰, -S(O)₂-NH₂, -S(O)₂-NR⁶-R⁰, -NR⁶-S(O)₂-R⁰, -R⁰⁰-O-CO-R⁰, -R⁰⁰-NR⁶-R⁰, -R⁰⁰-CN, -R⁰⁰-CONH₂, -R⁰⁰-CO-NR⁶-R⁰, -R⁰⁰-CO₂H, -R⁰⁰-CO₂-R⁰, -R⁰⁰-CO-R⁰, -R⁰⁰-NR⁶-CO-R⁰, -R⁰⁰-NR⁶-CO₂-R⁰, -R⁰⁰-O-CO-NR⁶-R⁰, cycloalkyl or nitrogen-containing saturated heterocyclic ring; wherein the nitrogen-containing saturated heterocyclic ring may be substituted with 1 to 2 substituents selected from the group consisting of lower alkyl, -OH, -O-R⁰, -NH₂, -NR⁶-R⁰ and oxo (=O);
R° is the same or different from each other and represents a lower alkyl which may be substituted with 1 or more substituents selected from the group consisting of-OH, -O-C₁₋₄ alkyl, -NH₂, -NR⁶-C₁₋₄ alkyl and halogen;
R⁶ is the same or different from one another and represents lower alkyl or H;
R⁰⁰ is the same or different from one another and represents lower alkylene;
p is 0, 1 or 2;
n is 0, 1 or 2;
m is 0 or 1;
R⁷ and R⁸ are the same or different from each other and each represents -H, -R°, halogen,
-OH, -O-R⁰, -NH₂, -NR⁶-R⁰, -NR⁶-CO-R⁰, -O-R⁰⁰-OH, -O-R⁰⁰-O-R⁰, cycloalkyl, nitrogen-containing saturated heterocyclic ring, or R⁷ and R⁸ may be taken together to form a group selected from the group consisting of oxo (=O), =N-OH, =N-OR⁰ and tetrahydropyranylidene, or R⁷ and R⁸ may be taken together to form a lower alkylene which may be interrupted by 1 to 2 groups selected from the group consisting of -O-, -S(O)ₚ-, -NR⁶- and -CONR⁶-, and may form a 3- to 8-membered ring together with the C atom to which they are attached.
Z is -NH-;
R³ is -H or R°; and
R⁴ and R⁵ are the same or different from each other and each represents -H, -R°, -CO₂-R⁰, -CO-R⁰, or R⁴ and R⁵ may be taken together to form a divalent group, which may form a 5-membered heterocyclic ring together with the -N-C-Z- group to which R⁴ and R⁵ are attached, wherein Z may further be -O- or S- and the 5-membered ring may be substituted with 1 or 2 substituents selected from lower alkyl, -OH, -O-R⁰, -NH₂, -NR⁶-R⁰, and oxo (=O).

5. The medicament for irritable bowel syndrome described in claim 4, wherein its active ingredient is a fluorene derivative or a salt thereof in which R³ is -H or R⁰ and R⁴ and R⁵ are -H or R⁰.

6. The medicament for irritable bowel syndrome described in claim 4, wherein its active ingredient is a fluorene derivative or a salt thereof in which every one of R³, R⁴ and R⁵ is -H.

7. The medicament for irritable bowel syndrome described in claim 6, wherein its active ingredient is a fluorene derivative or a salt thereof in which R⁷ and R⁸ are the same or different from each other and each represents -H, -R°, -OH, -O-R⁰, -O-R⁰⁰-OH or -O-R⁰⁰-O-R⁰, or R⁷ and R⁸ together form oxo group.

8. The medicament for irritable bowel syndrome described in claim 6, wherein its active ingredient is a fluorene derivative or a salt thereof in which R⁷ and R⁸ together form a "lower alkylene which may be discontinued with 1 or 2 divalent groups selected from the class consisting of -O-, -S(O)ₚ-, -NR⁶- and -CONR⁶-" and form a 3- to 8-membered ring together with the C atoms to which they are bonded.

9. The medicament for irritable bowel syndrome described in claim 4, wherein its active ingredient is a fluorene derivative or a salt thereof which is selected from the group consisting of N-(diaminomethylene)-9-hydroxy-9H-fluorene-2-carboxamide, 9-chloro-N-(diaminomethylene)-9H-fluorene-2-carboxamide, N-(diaminomethylene)-9-(hydroxyimino)-5-(hydroxymethyl)-9H-fluorene-2-carboxamide, 8-chloro-N-(diaminomethylene)-9-hydroxy-9H-fluorene-2-carboxamide, N-(diaminomethylene)-9-hydroxy-9-methyl-9H-fluorene-2-carboxamide, N-(diaminomethylene)-9-hydroxy-9-methyl-9H-fluorene-2-carboxamide (optically active substance A), N-(diaminomethylene)-9-hydroxy-9-methyl-9H-fluorene-2-carboxamide (optically active substance B), N-(diaminomethylene)spiro[1,3-dithiolane-2,9'-fluorene]-2'-carboxamide, N-(diaminomethylene)-4',5'-dihydro-3'H-spiro[fluorene-9,2'-furan]-2-carboxamide, N-(diaminomethylene)-4',5'-dihydro-3'H-spiro[fluorene-9,2'-furan]-2-carboxamide (optically active substance A), N-(diaminomethylene)-4',5'-dihydro-3'H-spiro[fluorene-9,2'-furan]-2-carboxamide (optically active substance B), N-(diaminomethylene)spiro[cyclopropane-1,9'-fluorene]-2'-carboxamide, N-(diaminomethylene)-9-methoxy-9-methyl-9H-fluorene-2-carboxamide, N-(diaminomethylene)-9-ethyl-9-methoxy-9H-fluorene-2-carboxamide, N-(diaminomethylene)-5-fluoro-9-hydroxy-9-methyl-9H-fluorene-2-carboxamide, N-(diaminomethylene)-5-fluoro-9-hydroxy-9-methyl-9H-fluorene-2-carboxamide (optically active substance A), N-(diaminomethylene)-5-fluoro-9-hydroxy-9-methyl-9H-fluorene-2-carboxamide (optically active substance B), N-(diaminomethylene)-5'-fluorospiro[1,3-dithiolane-2,9'-fluorene]-2'-carboxamide and N-(diaminomethylene)-5-fluoro-9-methoxy-9-methyl-9H-fluorene-2-carboxamide.

10. The medicament for irritable bowel syndrome described in claim 1, wherein the selective dual antagonist for 5-HT_{2B} and 5-HT₇ receptors comprises a) an antagonistic compound for 5-HT_{2B} receptor having selective binding affinity for 5-HT_{2B} receptor as a first component and b) an antagonistic compound for 5-HT₇ receptor having selective binding affinity for 5-HT₇ receptor as a second component.

11. A combination product for treating irritable bowel syndrome, which consists of a), as a first pharmaceutical preparation, a pharmaceutical preparation comprising an antagonistic compound for 5-HT_{2B} receptor having selective binding affinity for 5-HT_{2B} receptor as an active ingredient and b), as a second pharmaceutical preparation, a pharmaceutical preparation comprising an antagonistic compound for 5-HT₇ receptor having selective binding affinity for 5-HT₇ receptor as an active ingredient, wherein said first and second pharmaceutical preparations are administered simultaneously or separately.

12. Use of a selective dual antagonist for 5-HT_{2B} and 5-HT₇ receptors, for producing a medicament for irritable bowel syndrome.

13. Use of a "antagonistic compound for 5-HT_{2B} receptor having selective binding affinity for 5-HT_{2B} receptor", for producing a medicament for irritable bowel syndrome comprising a selective dual antagonist for 5-HT_{2B} and 5-HT₇ receptors as an active ingredient.

14. Use of a "antagonistic compound for 5-HT₇ receptor having selective binding affinity for 5-HT₇ receptor", for producing a medicament for irritable bowel syndrome comprising a selective dual antagonist for 5-HT_{2B} and 5-HT₇ receptors as an active ingredient.

15. A method for treating irritable bowel syndrome, which comprises administering an effective amount of a selective dual antagonist for 5-HT_{2B} and 5-HT₇ receptors to a patient.
